# EUROPEAN PATENT SPECIFICATION

(11) **EP 4 125 808 B1**
(45) Date of publication and mention of the grant of the patent: **14.10.2026**
(21) Application number: 21717180.0
(22) Date of filing: 01.04.2021
(51) Int. Cl.: A61K 9/00, A61K 38/06, A61K 31/191, A61K 33/30, A61P 3/10

(54) **NOVEL FORMULATIONS**
NEUARTIGE FORMULIERUNGEN
NOUVELLES FORMULATIONS

(30) Priority: 01.04.2020 GB 202004814
(43) Date of publication of application: 08.02.2023
(73) Proprietor: Arecor Limited, Little Chesterford Saffron Walden CB10 1XL (GB)
(72) Inventor: GERRING, David, Saffron Walden CB10 1XL (GB); HOWELL, Sarah, Saffron Walden CB10 1XL (GB); JEZEK, Jan, Saffron Walden CB10 1XL (GB); ZAKRZEWSKI, Leon, Saffron Walden CB10 1XL (GB)
(74) Representative: Sagittarius IP
(86) International application number: PCT/GB2021/050815
(87) International publication number: WO 2021/198694

(56) References cited:
- WO-A1-2018/060735

## Description

### FIELD OF THE INVENTION

This invention relates *inter alia* to rapid acting aqueous liquid formulations of fast acting insulin analogues for use in the treatment of subjects suffering from diabetes mellitus, especially Type 1 diabetes mellitus.

### BACKGROUND OF THE INVENTION

Diabetes mellitus ("diabetes") is a metabolic disorder associated with poor control of blood sugar levels leading to hypo or hyperglycaemia. Untreated diabetes can lead to serious microvascular and macrovascular complications including coronary artery disease, peripheral artery disease, stroke, diabetic nephropathy, neuropathy and retinopathy. The two main types of diabetes are (i) Type 1 diabetes resulting from the pancreas not producing insulin for which the usual treatment is insulin replacement therapy and (ii) Type 2 diabetes where patients either produce insufficient insulin or have insulin resistance and for which treatments include insulin sensitising agents (such as metformin or pioglitazone), traditional insulin secretagogues (such as sulfonylureas), SGLT2 inhibitors (such as dapagliflozin, canagliflozin and empagliflozin) which reduce glucose absorption in the kidneys and so promote glucose excretion, GLP-1 agonists (such as exenatide and dulaglutide) which stimulate insulin release from pancreatic beta cells and DPPIV inhibitors (such as sitagliptin or vildagliptin) which inhibit breakdown of GLP-1 leading to increased insulin secretion. Patients with Type 2 diabetes may eventually require insulin replacement therapy.

For patients requiring insulin replacement therapy, a range of therapeutic options are possible. The use of recombinant human insulin has in recent times been overtaken by use of insulin analogues which have modified properties, for example, are longer acting or faster acting than normal insulin. Thus, a common regimen for a patient involves receiving a long acting basal insulin supplemented by a rapid acting insulin around mealtimes.

Insulin is a peptide hormone formed of two chains (A chain and B chain, respectively 21 and 30 amino acids in length) linked via disulfide bridges. Insulin normally exists at neutral pH in the form of a hexamer, each hexamer comprising three dimers bound together by zinc ions. Histidine residues on the insulin are known to be involved in the interaction with the zinc ions. Insulin is stored in the body in the hexameric form but the monomer form is the active form. Traditionally, therapeutic compositions of insulin have also been formulated in hexameric form in the presence of zinc ions. Typically, there are approximately three zinc cations per one insulin hexamer. It has been appreciated that the hexameric form is absorbed from the injection site considerably more slowly than the monomeric and dimeric forms. Therefore, a faster onset of insulin action can be achieved if the hexameric form is destabilised allowing a more rapid dissociation of the zinc-bound hexamer into dimers and monomers in the subcutaneous space following injection. Three insulin analogues have been genetically engineered with this principle in mind. A first is insulin lispro (Humalog^{®}) in which residues 28 and 29 of the B chain (Pro and Lys respectively) are reversed, a second is insulin aspart (NovoRapid^{®}/NovoLog^{®}) in which residue 28 of the B chain, normally Pro, is replaced by Asp, and a third is insulin glulisine (Apidra^{®}) in which residue 3 of the B chain, normally Asn, is replaced by Lys and residue 29 of the B chain, normally Lys, is replaced by Glu.

Whilst the existing rapid acting insulin analogues can achieve a more rapid onset of action, it has been appreciated that even more rapid acting ("ultra rapid acting") insulins can be achieved by removing the zinc cations from insulin altogether. Unfortunately, the consequence of the hexamer dissociation is typically a considerable impairment in insulin stability both with respect to physical stability (e.g. stability to aggregation) and chemical stability (e.g. stability to deamidation). For example, monomeric insulin or insulin analogues having a rapid onset of action are known to aggregate and become physically unstable very rapidly because the formulation of insoluble aggregates proceeds via monomers of insulin. Various approaches to addressing this problem or otherwise increasing the speed of action of insulin have been described in the art:
US5,866,538 (Norup) describes insulin preparations of superior chemical stability comprising human insulin or an analogue or derivative thereof, glycerol and/or mannitol and 5 mM to 100 mM of a halogenide (e.g. NaCl).

US7,205,276 (Boderke) addresses the stability problems associated with preparing zinc-free formulations of insulin and insulin derivatives and analogues and describes an aqueous liquid formulation comprising at least one insulin derivative, at least one surfactant, optionally at least one preservative and optionally at least one of an isotonicizing agent, a buffer and an excipient, wherein the formulation is stable and free from or contains less than 0.4% (e.g. less than 0.2%) by weight of zinc based on the insulin content of the formulation. The preferred surfactant appears to be polysorbate 20 (polyoxyethylene (20) sorbitan monolaurate).

US2008/0194461 (Maggio) describes formulations of peptides and polypeptides including insulin which contain an alkyl glycoside, which component is said to reduce aggregation and immunogenicity.

WO2012/006283 (Pohl) describes formulations containing insulin together with a zinc chelator such as ethylenediaminetetraacetate (EDTA). Modulating the type and quantity of EDTA is said to change the insulin absorption profile. Calcium EDTA is the preferred form of EDTA since it is said to be associated with reduced pain at the injection site and is less likely to remove calcium from the body. Preferred formulations also contain citrate which is said to further enhance absorption and to improve the chemical stability of the formulation.

US2010/0227795 (Steiner) describes a composition comprising insulin, a dissociating agent such as citric acid or sodium citrate, and a zinc chelator such as EDTA wherein the formulation has a physiological pH and is a clear aqueous solution. The formulations are said to have improved stability and rapid onset of action.

WO2015/120457 (Wilson) describes stabilized ultra-rapid acting insulin formulations comprising insulin in combination with a zinc chelator such as EDTA, a dissolution/stabilization agent such as citric acid, a magnesium salt, a zinc compound and optionally additional excipients.

Further approaches to accelerating the absorption and effect of insulin through the use of specific accelerating additives have been described:
WO91/09617 (Jørgensen) reports that nicotinamide or nicotinic acid or a salt thereof increases the speed of absorption of insulin from aqueous preparations administered parenterally.

WO2010/149772 (Olsen) describes a formulation comprising insulin, a nicotinic compound and arginine. The presence of arginine is said to improve the chemical stability of the formulation. It would appear that an embodiment of this disclosure has been commercialised as Fiasp^{®}, which insulin product is claimed by the manufacturer to be faster acting than NovoRapid^{®}/NovoLog^{®}.

WO2015/171484 (Christe) describes rapid-acting formulations of insulin wherein onset of action and/or absorption of insulin is faster due to the presence of treprostinil.

US2013/0231281 (Soula) describes an aqueous solution composition comprising insulin or an insulin analogue and at least one oligosaccharide whose average degree of polymerisation is between 3 and 13 and whose polydispersity index is above 1.0, said oligosaccharide having partially substituted carboxyl functional groups, the unsubstituted carboxyl functional groups being salifiable. Such a formulation is said to be rapid acting.

WO2016/100042 (Eli Lilly and Company) describes a composition of human insulin or insulin analogue that includes specific concentrations of citrate, chloride, in some cases including the addition of sodium chloride, zinc and, optionally magnesium chloride and/or surfactant, said to have faster pharmacokinetic and/or pharmacodynamic action than commercial formulations of existing insulin analogue products.

WO2017/191464 (Arecor Limited) describes an aqueous liquid pharmaceutical formulation comprising insulin or an insulin analogue, ionic zinc, a chelating agent and polysorbate 80.

WO2018/060735 (Arecor Limited) describes an aqueous liquid pharmaceutical formulation comprising (i) an insulin compound, (ii) ionic zinc, (iii) a zinc binding species at a concentration of mM or more selected from species having a logK with respect to zinc ion binding in 5 the range 4.5-12.3 at 25 °C, and (iv) a non-ionic surfactant which is an alkyl glycoside; and wherein the formulation is substantially free of EDTA and any other zinc binding species having a logK with respect to zinc ion binding of more than 12.3 at 25 °C.

WO2018/203059 (Arecor Limited) describes an aqueous liquid pharmaceutical formulation comprising: (i) an insulin compound; (ii) ionic zinc; (iii) a zinc binding species at a concentration of 1 mM or more selected from species having a logK with respect to zinc ion binding in the range 4.5-10 at 25 °C; (iv) a zinc binding species selected from species having a logK with respect to zinc ion binding of more than 12.3 at 25 °C at a concentration of less than about 0.3 mM; and (v) a non-ionic surfactant.

It would be desirable if analogues or formulations of insulin were available which were ultra-rapid acting, thus more closely matching the activity of physiological insulin. There also remains a need in the art to provide further, and preferably improved, formulations of insulin and insulin analogues which are rapid acting and stable. The following invention results from a clinical study showing that the formulations described herein are indeed ultra-rapid acting and are significantly more rapid acting than both of the comparator formulations NovoRapid^{®} and Fiasp^{®} (of which two comparator formulations Fiasp^{®} seems to be the more rapid acting) This exceptional speed of action is not reported in any of the aforementioned disclosures.

### SUMMARY OF THE INVENTION

The invention is defined in the claims.

Described is an aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; for use in the treatment of a human subject suffering from diabetes mellitus by administration by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 14 mg/kg;
(ii) an area under the glucose infusion rate curve t=0-60 minutes (AUC_{GIR0-60}) of at least 130 mg/kg;
(iii) a time to onset of glucose lowering action (T_{GIRONSET}) of at most 21 minutes;
(iv) a time to 50% maximum glucose infusion rate in early (upward) part of the pharmacodynamic profile (T_{GIR50%MAX}) of at most 45 minutes;
(v) an area under the baseline corrected insulin concentration curve t=0-30 minutes (AUC_{INSBC0-30}) of at least 24 mU×h/L;
(vi) an area under the baseline corrected insulin concentration curve t=0-60 minutes (AUC_{INSBC0-60}) of at least 80 mU×h/L;
(vii) a time to initial onset of insulin exposure (T_{INSONSET}) of at most 4 minutes;
(viii) a time to maximum insulin concentration in the early (upward) part of the baseline corrected insulin curve (T_{INSMAX}) of at most 70 minutes;
(ix) a time to 50% maximum insulin concentration in the early (upward) part of the baseline corrected insulin curve (T_{INS50%MAX}) of at most 20 minutes; and
(x) a time to 50% maximum insulin concentration in the late (downward) part of the baseline corrected insulin curve (T_{INS50%MAXLATE}) of at most 205 minutes.

Also described is the use of an aqueous liquid pharmaceutical formulation in the manufacture of a medicament for the treatment of a human subject suffering from diabetes mellitus; said aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; wherein said treatment comprises administration of said formulation by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 14 mg/kg;
(ii) an area under the glucose infusion rate curve t=0-60 minutes (AUC_{GIR0-60}) of at least 130 mg/kg;
(iii) a time to onset of glucose lowering action (T_{GIRONSET}) of at most 21 minutes;
(iv) a time to 50% maximum glucose infusion rate in early (upward) part of the pharmacodynamic profile (T_{GIR50%MAX}) of at most 45 minutes;
(v) an area under the baseline corrected insulin concentration curve t=0-30 minutes (AUC_{INSBC0-30}) of at least 24 mU×h/L;
(vi) an area under the baseline corrected insulin concentration curve t=0-60 minutes (AUC_{INSBC0-60}) of at least 80 mU×h/L;
(vii) a time to initial onset of insulin exposure (T_{INSONSET}) of at most 4 minutes;
(viii) a time to maximum insulin concentration in the early (upward) part of the baseline corrected insulin curve (T_{INSMAX}) of at most 70 minutes;
(ix) a time to 50% maximum insulin concentration in the early (upward) part of the baseline corrected insulin curve (T_{INS50%MAX}) of at most 20 minutes; and
(x) a time to 50% maximum insulin concentration in the late (downward) part of the baseline corrected insulin curve (T_{INS50%MAXLATE}) of at most 205 minutes.

Further described is a method of treating a human subject suffering from diabetes mellitus by administration of an aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; wherein said administration is by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 14 mg/kg;
(ii) an area under the glucose infusion rate curve t=0-60 minutes (AUC_{GIR0-60}) of at least 130 mg/kg;
(iii) a time to onset of glucose lowering action (T_{GIRONSET}) of at most 21 minutes;
(iv) a time to 50% maximum glucose infusion rate in early (upward) part of the pharmacodynamic profile (T_{GIR50%MAX}) of at most 45 minutes;
(v) an area under the baseline corrected insulin concentration curve t=0-30 minutes (AUC_{INSBC0-30}) of at least 24 mU×h/L;
(vi) an area under the baseline corrected insulin concentration curve t=0-60 minutes (AUC_{INSBC0-60}) of at least 80 mU×h/L;
(vii) a time to initial onset of insulin exposure (T_{INSONSET}) of at most 4 minutes;
(viii) a time to maximum insulin concentration in the early (upward) part of the baseline corrected insulin curve (T_{INSMAX}) of at most 70 minutes;
(ix) a time to 50% maximum insulin concentration in the early (upward) part of the baseline corrected insulin curve (T_{INS50%MAX}) of at most 20 minutes; and
(x) a time to 50% maximum insulin concentration in the late (downward) part of the baseline corrected insulin curve (T_{INS50%MAXLATE}) of at most 205 minutes.

According to the present invention, there is provided an aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; for use in the treatment of a human subject suffering from diabetes mellitus by administration by subcutaneous injection or subcutaneous infusion as a prandial dose in the window between 20 minutes after the start of a meal and 30 minutes after the start of a meal, wherein the administration of 0.3 U/kg of the formulation leads to a time to onset of glucose lowering action (T_{GIRONSET}) of at most 50% of the corresponding value upon administration of Fiasp^{®}.

Described is the use of an aqueous liquid pharmaceutical formulation in the manufacture of a medicament for the treatment of a human subject suffering from diabetes mellitus; said aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; wherein said treatment comprises administration of said formulation by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 150%;
(ii) an area under the glucose infusion rate curve t=0-60 minutes (AUC_{GIR0-60}) of at least 150%;
(iii) a time to onset of glucose lowering action (T_{GIRONSET}) of at most 90%;
(iv) a time to 50% maximum glucose infusion rate in early (upward) part of the pharmacodynamic profile (T_{GIR50%MAX}) of at most 90%;
(v) an area under the baseline corrected insulin concentration curve t=0-30 minutes (AUC_{INSBC0-30}) of at least 130%;
(vi) an area under the baseline corrected insulin concentration curve t=0-60 minutes (AUC_{INSBC0-60}) of at least 125%;
(vii) a time to initial onset of insulin exposure (T_{INSONSET}) of at most 80%;
(viii) a time to maximum insulin concentration in the early (upward) part of the baseline corrected insulin curve (T_{INSMAX}) of at most 90%;
(ix) a time to 50% maximum insulin concentration in the early (upward) part of the baseline corrected insulin curve (T_{INS50%MAX}) of at most 85%; and
(x) a time to 50% maximum insulin concentration in the late (downward) part of the baseline corrected insulin curve (T_{INS50%MAXLATE}) of at most 93%; of the corresponding value upon administration of Fiasp^{®}.

Also described is a method of treating a human subject suffering from diabetes mellitus by administration of an aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; wherein said administration is by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 150%;
(ii) an area under the glucose infusion rate curve t=0-60 minutes (AUC_{GIR0-60}) of at least 150%;
(iii) a time to onset of glucose lowering action (T_{GIRONSET}) of at most 90%;
(iv) a time to 50% maximum glucose infusion rate in early (upward) part of the pharmacodynamic profile (T_{GIR50%MAX}) of at most 90%;
(v) an area under the baseline corrected insulin concentration curve t=0-30 minutes (AUC_{INSBC0-30}) of at least 130%;
(vi) an area under the baseline corrected insulin concentration curve t=0-60 minutes (AUC_{INSBC0-60}) of at least 125%;
(vii) a time to initial onset of insulin exposure (T_{INSONSET}) of at most 80%;
(viii) a time to maximum insulin concentration in the early (upward) part of the baseline corrected insulin curve (T_{INSMAX}) of at most 90%;
(ix) a time to 50% maximum insulin concentration in the early (upward) part of the baseline corrected insulin curve (T_{INS50%MAX}) of at most 85%; and
(x) a time to 50% maximum insulin concentration in the late (downward) part of the baseline corrected insulin curve (T_{INS50%MAXLATE}) of at most 93%; of the corresponding value upon administration of Fiasp^{®}.

Further described is an aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; for use in the treatment of a human subject suffering from diabetes mellitus by administration by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 5 mg/kg higher;
(ii) an area under the glucose infusion rate curve t=0-60 minutes (AUC_{GIR0-60}) of at least 45 mg/kg higher;
(iii) a time to onset of glucose lowering action (T_{GIRONSET}) of at least 2 minutes less;
(iv) a time to 50% maximum glucose infusion rate in early (upward) part of the pharmacodynamic profile (T_{GIR50%MAX}) of at least 5 minutes less;
(v) an area under the baseline corrected insulin concentration curve t=0-30 minutes (AUC_{INSBC0-30}) of at least 5 mU×h/L higher;
(vi) an area under the baseline corrected insulin concentration curve t=0-60 minutes (AUC_{INSBC0-60}) of at least 15 mU×h/L higher;
(vii) a time to initial onset of insulin exposure (T_{INSONSET}) of at least 1 minute less;
(viii) a time to maximum insulin concentration in the early (upward) part of the baseline corrected insulin curve (T_{INSMAX}) of at least 8 minutes less;
(ix) a time to 50% maximum insulin concentration in the early (upward) part of the baseline corrected insulin curve (T_{INS50%MAX}) of at least 4 minutes less; and
(x) a time to 50% maximum insulin concentration in the late (downward) part of the baseline corrected insulin curve (T_{INS50%MAXLATE}) of at least 16 minutes less; than that of the corresponding value upon administration of Fiasp^{®}.

Described is the use of an aqueous liquid pharmaceutical formulation in the manufacture of a medicament for the treatment of a human subject suffering from diabetes mellitus; said aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; wherein said treatment comprises administration of said formulation by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 5 mg/kg higher;
(ii) an area under the glucose infusion rate curve t=0-60 minutes (AUC_{GIR0-60}) of at least 45 mg/kg higher;
(iii) a time to onset of glucose lowering action (T_{GIRONSET}) of at least 2 minutes less;
(iv) a time to 50% maximum glucose infusion rate in early (upward) part of the pharmacodynamic profile (T_{GIR50%MAX}) of at least 5 minutes less;
(v) an area under the baseline corrected insulin concentration curve t=0-30 minutes (AUC_{INSBC0-30}) of at least 5 mU×h/L higher;
(vi) an area under the baseline corrected insulin concentration curve t=0-60 minutes (AUC_{INSBC0-60}) of at least 15 mU×h/L higher;
(vii) a time to initial onset of insulin exposure (T_{INSONSET}) of at least 1 minute less;
(viii) a time to maximum insulin concentration in the early (upward) part of the baseline corrected insulin curve (T_{INSMAX}) of at least 8 minutes less;
(ix) a time to 50% maximum insulin concentration in the early (upward) part of the baseline corrected insulin curve (T_{INS50%MAX}) of at least 4 minutes less; and
(x) a time to 50% maximum insulin concentration in the late (downward) part of the baseline corrected insulin curve (T_{INS50%MAXLATE}) of at least 16 minutes less; than that of the corresponding value upon administration of Fiasp^{®}.

Described is a method of treating a human subject suffering from diabetes mellitus by administration of an aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; wherein said administration is by subcutaneous injection or subcutaneous infusion at or close to a meal-time wherein the administration of 0.3 U/kg of the formulation leads to one or more of:
(i) an area under the glucose infusion rate curve t=0-30 minutes (AUC_{GIR0-30}) of at least 5 mg/kg higher;
(ii) an area under the glucose infusion rate curve t=0-60 minutes (AUC_{GIR0-60}) of at least 45 mg/kg higher;
(iii) a time to onset of glucose lowering action (T_{GIRONSET}) of at least 2 minutes less;
(iv) a time to 50% maximum glucose infusion rate in early (upward) part of the pharmacodynamic profile (T_{GIR50%MAX}) of at least 5 minutes less;
(v) an area under the baseline corrected insulin concentration curve t=0-30 minutes (AUC_{INSBC0-30}) of at least 5 mU×h/L higher;
(vi) an area under the baseline corrected insulin concentration curve t=0-60 minutes (AUC_{INSBC0-60}) of at least 15 mU×h/L higher;
(vii) a time to initial onset of insulin exposure (T_{INSONSET}) of at least 1 minute less;
(viii) a time to maximum insulin concentration in the early (upward) part of the baseline corrected insulin curve (T_{INSMAX}) of at least 8 minutes less;
(ix) a time to 50% maximum insulin concentration in the early (upward) part of the baseline corrected insulin curve (T_{INS50%MAX}) of at least 4 minutes less; and
(x) a time to 50% maximum insulin concentration in the late (downward) part of the baseline corrected insulin curve (T_{INS50%MAXLATE}) of at least 16 minutes less;
than that of the corresponding value upon administration of Fiasp^{®}.

The formulations administered in the use and methods provide insulin in a form which is ultra-rapid acting. Suitably said formulations also have good physical and chemical stability.

### DESCRIPTION OF THE SEQUENCE LISTING

SEQ ID NO: 1: A chain of human insulin
SEQ ID NO: 2: B chain of human insulin
SEQ ID NO: 3: B chain of insulin lispro
SEQ ID NO: 4: B chain of insulin aspart
SEQ ID NO: 5: B chain of insulin glulisine

### FIGURES

- Fig. 1:: Phase I pharmacodynamic profiles (Glucose Infusion Rate (GIR) as a function of time) of formulations AT500, Fiasp^{®} and NovoRapid^{®} (Example 2).
- Fig. 2:: Phase I pharmacokinetic profiles (baseline-corrected serum insulin aspart concentration as a function of time) of formulations AT500, Fiasp^{®} and NovoRapid^{®} (Example 2).

### DETAILED DESCRIPTION OF THE INVENTION

As used herein, "insulin" refers to native human insulin having an A chain and a B chain as set out in SEQ ID NOs. 1 and 2 and containing and connected by disulfide bridges as in the native molecule (Cys A6-Cys A11, Cys B7 to Cys A7 and Cys-B19-Cys A20). Insulin is suitably recombinant insulin. As an exception to the foregoing statement, a reference to "insulin" in respect of the pharmacodynamic and pharmacokinetic parameters of a formulation comprising a fast acting insulin analogue shall be a reference to the fast acting insulin analogue of that formulation.

A "fast acting insulin analogue" is an analogue of insulin which is an insulin receptor agonist and has a modified amino acid sequence, such as containing 1 or 2 amino acid changes in the sequence of the A or B chain (especially the B chain). Desirably such amino acid modifications are intended to reduce affinity of the molecule for zinc and thus increase speed of action. The fast acting insulin analogue has a speed of action which is greater than that of insulin. Suitably, the fast acting insulin analogue is defined as an insulin analogue which has a speed of action which is greater than that of native human insulin e.g. as measured using the Diabetic Pig Pharmacokinetic/Pharmacodynamic Model (see Examples, General Methods (a)). Exemplary fast acting insulin analogues include insulin lispro, insulin aspart and insulin glulisine. These forms of insulin have the human insulin A chain but variant B chains - see SEQ ID NOs. 3-5. Further fast acting analogues are described in EP0214826, EP0375437 and EP0678522.

As used herein, Fiasp^{®} is a formulation of insulin aspart having the composition stated in the Examples section.

As used herein, NovoRapid^{®} is a formulation of insulin aspart having the composition stated in the Examples section.

In one embodiment, the fast acting insulin analogue is insulin lispro. In another embodiment, it is insulin aspart. In another embodiment, it is insulin glulisine. Insulin analogues such as insulin glargine and insulin degludec are not fast acting insulin analogues.

The term "aqueous liquid pharmaceutical formulation", as used herein, refers to a formulation suitable for therapeutic use in which the aqueous component is or comprises water, preferably distilled water, deionized water, water for injection, sterile water for injection or bacteriostatic water for injection. The aqueous liquid pharmaceutical formulations of the invention are solution formulations in which all components are dissolved in water.

The term "prandial" as used herein to describe an aqueous liquid pharmaceutical formulation, refers to a formulation which is administered to a patient before or during a mealtime.

The concentration of fast acting insulin analogue in the formulation will typically be in the range 50-200 U/ml. An exemplary formulation contains fast acting insulin analogue at a concentration of around 100 U/ml (around 3.6 mg/ml) e.g. 100 U/ml. A further exemplary formulation contains fast acting insulin analogue at a concentration of around 200 U/ml (around 7.2 mg/ml) e.g. 200 U/ml. Further exemplary formulation contains fast acting insulin analogue at a concentration of around 70-130 U/ml e.g. 80-120 U/ml e.g. 90-110 U/ml e.g. 95-105 U/ml.

"U/ml" as used herein describes the concentration of fast acting insulin analogue in terms of a unit per volume, wherein "U" is the international unit of insulin activity (see e.g. European Pharmacopoeia 5.0, Human Insulin, pp 1800-1802).

The formulations for use according to the invention contain ionic zinc i.e. Zn²⁺ ions. The source of the ionic zinc will typically be a water-soluble zinc salt such as ZnCl₂, ZnO, ZnSO₄, Zn(NO₃)₂ or Zn(acetate)₂ and most suitably ZnCl₂ or ZnO.

The concentration of the ionic zinc in the formulation will typically be more than 0.05% e.g. more than 0.1% e.g. more than 0.2%, more than 0.3% or more than 0.4% by weight of zinc based on the weight of fast acting insulin analogue in the formulation. Thus, the concentration of the ionic zinc in the formulation may be more than 0.5% by weight of zinc based on the weight fast acting insulin analogue in the formulation, for example 0.5-1%, e.g. 0.5-0.75%, e.g. 0.5-0.6% by weight of zinc based on the weight of fast acting insulin analogue in the formulation. For the purpose of the calculation the weight of the counter ion to zinc is excluded. The concentration of the ionic zinc in the formulation will (for example, for a formulation containing 100 U/ml of fast acting insulin analogue) typically be more than 0.015 mM e.g. more than 0.03 mM e.g. more than 0.06 mM, more than 0.09 mM or more than 0.12 mM. Thus, the concentration of the ionic zinc in the formulation may be more than 0.15 mM, for example 0.15-0.60 mM, e.g. 0.20-0.45 mM, e.g. 0.25-0.35 mM.

The formulations for use according to the invention contain citrate. Citrate typically serves as a zinc binding species. Citrate has a logK metal binding stability constant with respect to zinc ion binding of 4.93 at 25 °C, as listed in the National Institute of Standards and Technology reference database 46 (Critically Selected Stability Constants of Metal Complexes). The presence of citrate in the formulation (without being limited by theory, believed to be due to its activity as a zinc binding species) contributes to the speed of action.

The concentration of citrate will typically be in the range 1-100 mM e.g. 1-50 mM, 5-50 mM, 10-50 mM, 10-30 mM, especially 20-30 mM. The concentration of citrate can be adjusted according to the particular concentration of fast acting insulin analogue present in the composition in order to provide the desired accelerating effect.

For 100 U/ml fast acting insulin analogue formulations, the concentration of citrate is suitably 10-50 mM e.g. 10-30 mM, more preferably around 20 mM (e.g. 22 mM). An alternative suitable range is 30-50 e.g. 35-50 e.g. around 44 mM.

Suitably, the molar ratio of ionic zinc to citrate is 1:10-1:500 e.g. 1:20-1:500 e.g. 1:20-1:100 or 1:40-1:250, e.g. 1:40-1:90 or 1:60-1:200, e.g. 1:60-1:80. The following ranges are particularly of interest: 1:10-1:500 e.g. 1:10-1:200 e.g. 1:10 to 1:100 e.g. 1:25 to 1:100 e.g. 1:50-1:100, e.g. 1:60-1:80 (especially for 100 U/ml fast acting insulin analogue formulations). For 200 U/ml fast acting insulin analogue formulations the preferred ratio is slightly smaller e.g. 1:25 to 1:50.

For example, a formulation containing 100 U/ml of fast acting insulin analogue may contain around 0.3 mM of ionic zinc (i.e. around 19.7 µg/ml of ionic zinc, i.e. around 0.54% by weight of zinc based on the weight of fast acting insulin analogue in the formulation) and around 15-30 mM e.g. 20-30 mM of citrate.

In one embodiment, the ratio of fast acting insulin analogue concentration (U/ml) to citrate (mM) in the formulation is in the range 100:1 to 2:1 e.g. 50:1 to 2:1 e.g. 40:1 to 2:1.

The formulations for use according to the invention are substantially free of zinc binding species which have a logK metal binding stability constant with respect to zinc binding of more than 12.3 as determined at 25 °C, suitably, are free of said zinc binding species. Specifically, the formulations of the invention are substantially free e.g. free of EDTA (logK = 14.5). Further examples of zinc binding species which have a logK metal binding stability constant with respect to zinc binding of more than 12.3 to be avoided include EGTA (logK = 12.6). In general formulations for use according to the invention will be substantially free of tetradentate ligands or ligands of higher denticity. In an embodiment, the formulations for use according to the invention are also substantially free of zinc binding species which have a logK metal binding stability constant with respect to zinc ion binding of 10-12.3 as determined at 25 °C. "Substantially free" means that the concentration of zinc binding species which have a logK metal binding stability constant with respect to zinc binding as specified (such as EDTA) is less than 0.1 mM, such as less than 0.05 mM, such as less than 0.04 mM or less than 0.01 mM.

Citrate may be introduced into the aqueous formulations for use according to the invention in the form of a salt of citric acid, such as a sodium salt (e.g. trisodium citrate). Alternatively, citrate can be introduced in the form of the acid (citric acid) with subsequent adjustment of pH to the required level. Thus, in an embodiment, the source of the citrate is citric acid.

Suitably, the formulations for use according to the invention contain a non-ionic surfactant.

A suitable class of non-ionic surfactants is the alkyl glycosides, especially dodecyl maltoside. In one embodiment, the alkyl glycoside is decyl glucopyranoside. Other alkyl glycosides include dodecyl glucoside, octyl glucoside, octyl maltoside, decyl glucoside, decyl maltoside, tridecyl glucoside, tridecyl maltoside, tetradecyl glucoside, tetradecyl maltoside, hexadecyl glucoside, hexadecyl maltoside, sucrose monooctanoate, sucrose mono decanoate, sucrose monododecanoate, sucrose monotridecanoate, sucrose monotetradecanoate and sucrose monohexadecanoate.

Another suitable class of non-ionic surfactants is the polysorbates (fatty acid esters of ethoxylated sorbitan), such as polysorbate 20 or polysorbate 80. Polysorbate 20 is a mono ester formed from lauric acid and polyoxyethylene (20) sorbitan in which the number 20 indicates the number of oxyethylene groups in the molecule. Polysorbate 80 is a mono ester formed from oleic acid and polyoxyethylene (20) sorbitan in which the number 20 indicates the number of oxyethylene groups in the molecule. Polysorbate 20 is known under a range of brand names including in particular Tween 20, and also Alkest TW 20. Polysorbate 80 is known under a range of brand names including in particular Tween 80, and also Alkest TW 80. Other suitable polysorbates include polysorbate 40 and polysorbate 60. In an embodiment, the non-ionic surfactant is other than polysorbate 80. In one embodiment, the non-ionic surfactant is other than polysorbate 20.

Another suitable class of non-ionic surfactants is block copolymers of polyethylene glycol and polypropylene glycol, also known as poloxamers, especially poloxamer 188, poloxamer 407, poloxamer 171 and poloxamer 185. Poloxamers are also known under brand names Pluronics or Koliphors. For example, poloxamer 188 is marketed as Pluronic F-68.

Another suitable class of non-ionic surfactants is alkyl ethers of polyethylene glycol, especially those known under a brand name Brij, such as selected from polyethylene glycol (2) hexadecyl ether (Brij 52), polyethylene glycol (2) oleyl ether (Brij 93) and polyethylene glycol (2) dodecyl ether (Brij L4). Other suitable Brij surfactants include polyethylene glycol (4) lauryl ether (Brij 30), polyethylene glycol (10) lauryl ether (Brij 35), polyethylene glycol (20) hexadecyl ether (Brij 58) and polyethylene glycol (10) stearyl ether (Brij 78).

Another suitable class of non-ionic surfactants are alkylphenyl ethers of polyethylene glycol, especially 4-(1,1,3,3-tetramethylbutyl)phenyl-polyethylene glycol, also known under a brand name Triton X-100.

Particularly suitable are non-ionic surfactants with molecular weight of less than 1000 g/mole, especially less than 600 g/mole, such as 4-(1,1,3,3-tetramethylbutyl)phenyl-polyethylene glycol (Triton X-100) (647 g/mole), dodecyl maltoside (511 g/mole), octyl glucoside (292 g/mole), polyethylene glycol (2) dodecyl ether (Brij L4) (362 g/mole), polyethylene glycol (2) oleyl ether (Brij 93) (357 g/mole) and polyethylene glycol (2) hexadecyl ether (Brij 52) (330 g/mole).

The concentration of a non-ionic surfactant in the formulation will typically be in the range 1-1000 µg/ml, e.g. 5-500 µg/ml, e.g. 10-200 µg/ml, such as 10-100 µg/ml especially around 50 µg/ml. In one embodiment, the non-ionic surfactant is present at a concentration of 10-400 µg/ml e.g. 20-400 µg/ml, 50-400 µg/ml, 10-300 µg/ml, 20-300 µg/ml, 50-300 µg/ml, 10-200 µg/ml, 20-200 µg/ml or 50-200 µg/ml.

In one embodiment, the formulations for use according to the invention contain a non-ionic surfactant which is an alkyl glycoside, especially dodecyl maltoside. In another embodiment, the alkyl glycoside is decyl glucopyranoside. Suitably, the alkyl glycoside is present at a concentration of 10-400 µg/ml e.g. 20-400 µg/ml, 50-400 µg/ml, 10-300 µg/ml, 20-300 µg/ml, 50-300 µg/ml, 10-200 µg/ml, 20-200 µg/ml, 50-200 µg/ml, 10-100 µg/ml, 20-100 µg/ml or 50-100 µg/ml e.g. around 50 µg/ml.

In some embodiments the alkyl glycoside is dodecyl maltoside, and the dodecyl maltoside is present at a concentration of 10-400 µg/ml e.g. 20-400 µg/ml, 50-400 µg/ml, 10-300 µg/ml, 20-300 µg/ml, 50-300 µg/ml, 10-200 µg/ml, 20-200 µg/ml, 50-200 µg/ml, 10-100 µg/ml, 20-100 µg/ml or 50-100 µg/ml e.g. around 50 µg/ml.

In one embodiment, the concentration of fast acting insulin analogue is about 50 to about 200 U/ml and the formulation further contains a non-ionic surfactant such as alkyl glycoside (e.g. dodecyl maltoside) at a concentration of 50-100 µg/ml.

Suitably the pH of the aqueous formulations for use according to the invention is in the range 5.5-9.0 especially 6.5-8.0 e.g. 7.0-8.0 e.g. 7.0-7.8. e.g. 7.0-7.5. In order to minimise injection pain, the pH is preferably close to physiological pH (around pH 7.4). Another pH of interest is 7.6-8.0 e.g. around 7.8. An additional pH range of interest is 7.4-7.8, e.g. around 7.4 or around 7.8. Another possible pH is around 7.6.

Suitably, the composition for use according to the invention comprises a buffer (e.g. one or more buffers) in order to stabilise the pH of the formulation, which can also be selected to enhance the stability of the fast acting insulin analogue. In one embodiment, a buffer is selected to have a pKₐ close to the pH of the composition; for example, histidine is suitably employed as a buffer when the pH of the composition is in the range 5.0-7.0. Such a buffer may be employed in a concentration of 0.5-20 mM e.g. 2-5 mM. As another example, phosphate e.g. sodium phosphate is suitably employed as a buffer when the pH of the composition is in the range 6.1-8.1. Such a buffer may be employed in a concentration of 0.5-20 mM e.g. 2-5 mM, e.g. 2 mM. Alternatively, in another embodiment, the formulation is further stabilised as disclosed in WO2008/084237 which describes a formulation comprising a protein and one or more additives, characterised in that the system is substantially free of a conventional buffer, i.e. a compound with an ionisable group having a pKₐ within 1 unit of the pH of the formulation at the intended temperature range of storage of the composition, such as 25 °C. In this embodiment, the pH of the formulation is set to a value at which the formulation has maximum measurable stability with respect to pH; the one or more additives (displaced buffers) are capable of exchanging protons with the fast acting insulin analogue and have pKₐ values at least 1 unit more or less than the pH of the formulation at the intended temperature range of storage of the formulation. The additives may have ionisable groups having pKₐ between 1 to 5 pH units, preferably between 1 to 3 pH units, most preferably from 1.5 to 2.5 pH units, of the pH of the aqueous formulation at the intended temperature range of storage of the composition (e.g. 25 °C). Such additives may typically be employed at a concentration of 0.5-10 mM e.g. 2-5 mM.

The aqueous formulations for use according to the present invention cover a wide range of osmolarity, including hypotonic, isotonic and hypertonic compositions. Preferably, the formulations of the invention are substantially isotonic. Suitably the osmolarity of the formulation is selected to minimize pain according to the route of administration e.g. upon injection. Preferred formulations have an osmolarity in the range of about 200 to about 500 mOsm/L. Preferably, the osmolarity is in the range of about 250 to about 350 mOsm/L. More preferably, the osmolarity is about 300 mOsm/L.

Tonicity of the formulation may be adjusted with a tonicity modifying agent (e.g. one or more tonicity modifying agents). Tonicity modifying agents may be charged or uncharged. Examples of charged tonicity modifying agents include salts such as a combination of sodium, potassium, magnesium or calcium ions, with chloride, sulfate, carbonate, sulfite, nitrate, lactate, succinate, acetate or maleate ions (especially sodium chloride or sodium sulphate, particularly sodium chloride). The fast acting insulin analogue formulations for use according to the invention may contain a residual NaCl concentration of 2-4 mM as a result of the use of standard acidification and subsequent neutralization steps employed in preparing insulin formulations. Amino acids such as arginine, glycine or histidine may also be used for this purpose. When present in the formulation, a charged tonicity modifying agent (e.g. NaCl) is preferably used at a concentration of 100-300 mM, e.g. around 150 mM. In one embodiment, the formulation of the invention comprises >60 mM chloride e.g. sodium chloride, for example >65 mM, >75 mM, >80 mM, >90 mM, >100 mM, >120 mM or >140 mM, such as >60-300 mM, >60-200 mM, 50-175 mM, 75-300 mM, 75-200 mM or 75-175 mM. In one embodiment, the formulation further contains a charged tonicity modifying agent which is sodium chloride at a concentration of 100-300 mM, e.g. around 150 mM.

In one embodiment, the fast acting insulin analogue is insulin lispro at a concentration of 50-200 U/ml such as 100 U/ml, and the formulation further contains a charged tonicity modifying agent which is sodium chloride at a concentration of 100-300 mM, e.g. around 150 mM.

In one embodiment, the fast acting insulin analogue is insulin aspart at a concentration of 50-200 U/ml such as 100 U/ml, and the formulation further contains a charged tonicity modifying agent which is sodium chloride at a concentration of 100-300 mM, e.g. around 150 mM.

In one embodiment, the fast acting insulin analogue is insulin glulisine at a concentration of 50-200 U/ml such as 100 U/ml, and the formulation further contains a charged tonicity modifying agent which is sodium chloride at a concentration of 100-300 mM, e.g. around 150 mM.

Examples of uncharged tonicity modifying agents include sugars, sugar alcohols and other polyols, such as trehalose, sucrose, mannitol, glycerol, 1,2-propanediol, raffinose, lactose, dextrose, sorbitol or lactitol (especially trehalose, mannitol, glycerol or 1,2-propanediol, particularly glycerol). When present in the formulation, an uncharged tonicity modifying agent is preferably used at a concentration of 100-500 mM e.g. 100-300 mM, e.g. 150-200 e.g. around 174 mM. In one embodiment, the formulation contains an uncharged tonicity modifying agent which is used at a concentration of 100-300 mM, e.g. 150-200 mM, 170-180 mM or around 174 mM. In one embodiment, the formulation further contains an uncharged tonicity modifying agent which is glycerol at a concentration of 100-300 mM, e.g. 150-200 mM, 170-180 mM or around 174 mM.

The ionic strength of a formulation may be calculated according to the formula la: $I = 0.5 \times {\sum }_{X = 1}^{n} {c}_{x} { z}_{x}^{2}$ in which cₓ is molar concentration of ion x (mol L⁻¹), zₓ is the absolute value of the charge of ion x and the sum covers all ions (n) present in the composition. The contribution of the fast acting insulin analogue itself should be ignored for the purposes of the calculation. The contribution of the citrate should be ignored for the purposes of the calculation. Suitably, the contribution of the ionic zinc should be included for the purposes of the calculation. For zwitterions, the absolute value of the charge is the total charge excluding polarity, e.g. for glycine the possible ions have absolute charge of 0, 1 or 2 and for aspartate the possible ions have absolute charge of 0, 1, 2 or 3.

In general, the ionic strength of the formulation is suitably in the range of around 1 mM up to around 500 mM e.g. 1-500 mM e.g. 1-400 mM e.g. 1-300 mM.

An exemplary formulation of interest comprises (i) a fast acting insulin analogue, such as insulin aspart, e.g. at a concentration of around 100 U/ml, (ii) ionic zinc, e.g. as ZnCl₂, e.g. at a zinc concentration of around 0.5-1% by weight of zinc based on the weight of fast acting insulin analogue in the formulation, for example around 0.25-0.35 mM e.g. around 0.3 mM (iii) a non-ionic surfactant e.g. an alkyl glycoside such a dodecyl maltoside e.g. at a concentration of around 50-200 ug/ml e.g. around 50 ug/ml; and (iv) a charged tonicity modifier e.g. sodium chloride e.g. at a concentration of around 100-300 mM e.g. around 150 mM and which formulation has a pH of around 7.0-8.0 e.g. around 7.4-7.8 e.g. around 7.4 or around 7.8.

Another exemplary formulation of interest comprises (i) a fast acting insulin analogue, such as insulin aspart, e.g. at a concentration of around 100 U/ml, (ii) ionic zinc, e.g. as ZnCl₂, e.g. at a zinc concentration of around 0.5-1% by weight of zinc based on the weight of fast acting insulin analogue in the formulation, for example around 0.25-0.35 mM e.g. around 0.3 mM (iii) a non-ionic surfactant e.g. an alkyl glycoside such a dodecyl maltoside e.g. at a concentration of around 50-200 ug/ml e.g. around 50 ug/ml; and (iv) an uncharged tonicity modifier e.g. selected from sugars, sugar alcohols and other polyols, e.g. at a concentration of around 100-500 mM e.g. 100-300 mM, e.g. 150-200 e.g. around 174 mM and which formulation has a pH of around 7.0-8.0 e.g. around 7.4-7.8 e.g. around 7.4 or around 7.8.

The formulations for use according to the invention can optionally include a preservative (e.g. one or more preservatives), preferably phenol, m-cresol, chlorocresol, benzyl alcohol, propylparaben, methylparaben, benzalkonium chloride or benzethonium chloride. In one embodiment, the formulation includes phenol or m-cresol. In one embodiment, a mixture of preservatives is employed e.g. phenol and m-cresol.

The formulations for use according to the invention may optionally comprise nicotinamide. The presence of nicotinamide may further increase the speed of onset of action of the fast acting insulin analogue formulated in compositions of the invention. Suitably, the concentration of nicotinamide is in the range 10-150 mM, preferably in the range 20-100 mM, such as around 80 mM.

The formulations for use according to the invention may optionally comprise nicotinic acid or a salt thereof. The presence of nicotinic acid or a salt thereof may also further increase the speed of onset of action of the fast acting insulin analogue formulated in compositions of the invention. Suitably, the concentration of nicotinic acid or a salt thereof is in the range 10-250 mM, preferably in the range 50-200 mM, such as around 170 mM. Example salts include metal salts such as sodium, potassium and magnesium salts.

Typically, one of nicotinamide and nicotinic acid (or as salt thereof) may be included in the formulation but not both.

The formulations for use according to the invention may optionally comprise treprostinil or a salt thereof. The presence of the treprostinil may further increase the speed of onset of action of the fast acting insulin analogue formulated in compositions of the invention. Suitably, the concentration of treprostinil in the formulation is in the range of 0.1-12 µg/ml e.g. 0.1-10 µg/ml, 0.1-9 µg/ml, 0.1-8 µg/ml, 0.1-7 µg/ml, 0.1-6 µg/ml, 0.1-5 µg/ml, 0.1-4 µg/ml, 0.1-3 µg/ml, 0.1-2 µg/ml, 0.5-2 µg/ml e.g. about 1 µg/ml.

In one embodiment, the formulation does not contain a vasodilator. In a further embodiment, the formulation does not contain treprostinil, nicotinamide, nicotinic acid or a salt thereof.

Formulations for use according to the invention may optionally include other beneficial components including stabilising agents. For example, amino acids such as arginine or proline may be included which may have stabilising properties. Thus, in one embodiment, the formulations of the invention comprise arginine.

In an embodiment of the invention the formulations are free of acids selected from glutamic acid, ascorbic acid, succinic acid, aspartic acid, maleic acid, fumaric acid, adipic acid and acetic acid and are also free from the corresponding ionic forms of these acids.

In an embodiment of the invention the formulations are free of arginine.

In an embodiment of the invention the formulations are free of protamine and protamine salts.

In an embodiment of the invention the formulations are free of magnesium ions.

The addition of magnesium ions e.g. in the form of magnesium chloride can provide a stabilising effect. Thus, in an embodiment of the invention a formulation contains magnesium ions e.g. MgCl₂.

In an embodiment of the invention the formulations are free of calcium ions.

Formulations for use according to the invention may further comprise an additional therapeutically active agent (an "active agent"), in particular an agent of use in the treatment of diabetes (i.e. in addition to the fast acting insulin analogue) e.g. an amylin analogue or a GLP-1 agonist. In one embodiment, the formulation further comprises an amylin analogue such as pramlintide, suitably at a concentration of 0.1-10 mg/ml e.g. 0.2-6 mg/ml. In one embodiment, the formulation further comprises a GLP-1 agonist such as liraglutide, dulaglutide, albiglutide, exenatide, semaglutide or lixisenatide, suitably at a concentration of 10 µg/ml to 50 mg/ml e.g. 200 µg/ml to 10 mg/ml or 1-10 mg/ml. Formulations for use according to the invention may further comprise a long acting insulin such as insulin glargine or insulin degludec, suitably at a concentration of 50-1000 U/ml e.g. 100-500 U/ml or 100-200 U/ml.

Suitably the formulations for use according to the invention are sufficiently stable that the concentration of high molecular weight species remains low upon extended storage. The term "high molecular weight species" as used herein, refers to any irreversibly formed component of the protein content which has an apparent molecular weight at least about double the molecular weight of the parent fast acting insulin analogue, as detected by a suitable analytical method, such as size-exclusion chromatography. That is, high molecular weight species are multimeric aggregates of the parent fast acting insulin analogue. The multimeric aggregates may comprise the parent fast acting insulin analogue with considerably altered conformation or they may be an assembly of the parent fast acting insulin analogue units in the native or near-native conformation. The determination of high molecular weight species can be done using methods known in the art, including size exclusion chromatography, electrophoresis, analytical ultracentrifugation, light scattering, dynamic light scattering, static light scattering and field flow fractionation.

Suitably the formulations for use according to the invention are sufficiently stable that they remain substantially free of visible particles after storage at 25°C for at least 3 months e.g. at least 6 months and/or substantially free of visible particles after storage at 2-8°C for at least 9 months e.g. at least 12 months. Visible particles are suitably detected using the 2.9.20. European Pharmacopoeia Monograph (Particulate Contamination: Visible Particles). For example, a formulation is substantially free of visible particles if it has a Visual score according to Visual Assessment Method of 1 or 2 according to the definition given in Example 3.

Suitably the formulations for use according to the invention are sufficiently stable that the concentration of high molecular weight species remains low upon extended storage. High molecular weight species are suitably detected by SEC (see Example 3).

Suitably the formulation for use according to the invention comprises no more than 1% (by weight of total protein), preferably no more than 0.8% high molecular weight species after storage at 25°C for six months and/or the formulation of the invention comprises no more than 0.3% (by weight of total protein), preferably no more than 0.25% high molecular weight species after storage at 2-8°C for twelve months.

Suitably the formulations for use according to the invention are sufficiently stable that the concentration of related species remains low upon extended storage. The term "related species" as used herein, refers to any component of the protein content formed by a chemical modification of the parent fast acting insulin analogue, particularly desamido or cyclic imide forms of the insulin analogue. For example, these include B28isoAsp, A21Asp, B3Asp and B3isoAsp in the case of insulin aspart. Related species are suitably detected by RP-HPLC (see Example 3).

Suitably the formulation for use according to the invention comprises no more than 6% (by weight of total protein), preferably no more than 9% (by weight of total protein) of related species (e.g. B28isoAsp, A21Asp, B3Asp and B3isoAsp in the case of insulin aspart) after storage at 25°C for six months and/or 5% (by weight of total protein) of related species (e.g. B28isoAsp, A21Asp, B3Asp and B3isoAsp in the case of insulin aspart) after storage at 25°C for three months and/or the formulation of the invention comprises no more than 2% (by weight of total protein), preferably no more than 1.5% of related species (e.g. B28isoAsp, A21Asp, B3Asp and B3isoAsp in the case of insulin aspart) after storage at 2-8°C for twelve months.

### Pharmacodynamic parameters

In an embodiment, the area under the glucose infusion rate curve t=0-30 minutes (AUC_{GIR0-30}) following administration of 0.3 U/kg of the formulation is at least 14 mg/kg. Suitably the area under the glucose infusion rate curve t=0-30 minutes (AUC_{GIR0-30}) is at least 16 mg/kg e.g. at least 18 mg/kg e.g. at least 21 mg/kg e.g. at least 24 mg/kg e.g. at least 27 mg/kg e.g. at least 30 mg/kg e.g. at least 33 mg/kg e.g. at least 36 mg/kg e.g. at least 39 mg/kg. Typically, the area under the glucose infusion rate curve t=0-30 minutes (AUC_{GIR0-30}) is less than 48 mg/kg e.g. less than 44 mg/kg. An exemplary range is 14-48 mg/kg e.g. 33-48 mg/kg e.g. 36-44 mg/kg. The effect will for example be shown as a median effect in a group of at least 19 subjects.

In an embodiment, the area under the glucose infusion rate curve t=0-30 minutes (AUC_{GIR0-30}) following administration of 0.3 U/kg of the formulation is at least 150% of the corresponding value of Fiasp^{®}. Suitably the area under the glucose infusion rate curve t=0-30 minutes (AUC_{GIR0-30}) is at least 200% e.g. at least 250% e.g. at 300% e.g. at least 350% e.g. at least 400% of the corresponding value of Fiasp^{®}. Typically, the area under the glucose infusion rate curve t=0-30 minutes (AUC_{GIR0-30}) is at most 500% e.g. at most 450% of the corresponding value of Fiasp^{®}. An exemplary range is 150-500% e.g. 350-500% e.g. 400-450% of the corresponding value of Fiasp^{®}. The effect will for example be shown as a median effect in a group of at least 19 subjects per treatment drug.

In an embodiment, the area under the glucose infusion rate curve t=0-30 minutes (AUC_{GIR0-30}) following administration of 0.3 U/kg of the formulation is at least 5 mg/kg higher than that of the corresponding value of Fiasp^{®}. Suitably the area under the glucose infusion rate curve t=0-30 minutes (AUC_{GIR0-30}) is at least 10 mg/kg, e.g. at least 12 mg/kg, e.g. at least 15 mg/kg, e.g. at least 18 mg/kg higher e.g. at least 22 mg/kg higher e.g. at least 26 mg/kg higher than that of the corresponding value of Fiasp^{®}. Typically, the area under the glucose infusion rate curve t=0-30 minutes (AUC_{GIR0-30}) is at most 38 mg/kg e.g. at most 34 mg/kg higher than the corresponding value of Fiasp^{®}. An exemplary range is 5-38 mg/kg e.g. 18-38 mg/kg e.g. 22-34 mg/kg higher than the corresponding value of Fiasp^{®}. The effect will for example be shown as a median effect in a group of at least 19 subjects per treatment drug.

In an embodiment, the area under the glucose infusion rate curve t=0-60 minutes (AUC_{GIR0-60}) following administration of 0.3 U/kg of the formulation is at least 130 mg/kg. Suitably the area under the glucose infusion rate curve t=0-60 minutes (AUC_{GIR0-60}) e.g. at least 140 mg/kg, e.g. at least 160 mg/kg, e.g. at least 180 mg/kg, e.g. at least 190 mg/kg, e.g. at least 200 mg/kg, e.g. at least 210 mg/kg. Typically, the area under the glucose infusion rate curve t=0-60 minutes (AUC_{GIR0-60}) is less than 250 mg/kg e.g. less than 230 mg/kg. An exemplary range is 130-250 mg/kg e.g. 180-250 mg/kg e.g. 190-230 mg/kg. The effect will for example be shown as a median effect in a group of at least 19 subjects.

In an embodiment, the area under the glucose infusion rate curve t=0-60 minutes (AUC_{GIR0-60}) following administration of 0.3 U/kg of the formulation is at least 150% than that of the corresponding value of Fiasp^{®}. Suitably the area under the glucose infusion rate curve t=0-60 minutes (AUC_{GIR0-60}) is at least 160%, e.g. at least 170%, e.g. at least 180%, e.g. at least 190%, e.g. at least 200%, e.g. at least 210%, e.g. at least 220%, e.g. at least 230% of that of the corresponding value of Fiasp^{®}. Typically, the area under the glucose infusion rate curve t=0-60 minutes (AUC_{GIR0-60}) is at most 300% e.g. at most 260% of the corresponding value of Fiasp^{®}. An exemplary range is 150-300% e.g. 180-300% e.g. 200-260% of the corresponding value of Fiasp^{®}. The effect will for example be shown as a median effect in a group of at least 19 subjects per treatment drug.

In an embodiment, the area under the glucose infusion rate curve t=0-60 minutes (AUC_{GIR0-60}) following administration of 0.3 U/kg of the formulation is at least 45 mg/kg higher than that of the corresponding value of Fiasp^{®}. Suitably the area under the glucose infusion rate curve t=0-60 minutes (AUC_{GIR0-60}) is at least 50 mg/kg, e.g. at least 60 mg/kg, e.g. at least 70 mg/kg, e.g. at least 80 mg/kg higher e.g. at least 100 mg/kg higher than that of the corresponding value of Fiasp^{®}. Typically, the area under the glucose infusion rate curve t=0-60 minutes (AUC_{GIR0-60}) is at most 160 mg/kg e.g. at most 140 mg/kg higher than the corresponding value of Fiasp^{®}. An exemplary range is 45-160 mg/kg e.g. 70-160 mg/kg e.g. 80-140 mg/kg higher than the corresponding value of Fiasp^{®}. The effect will for example be shown as a median effect in a group of at least 19 subjects per treatment drug.

In an embodiment, the time to onset of glucose lowering action (T_{GIRONSET}) following administration of 0.3 U/kg of the formulation is at most 21 minutes. Suitably the time to onset of glucose lowering action occurs is at most 20 minutes administration e.g. less than 19 minutes e.g. less than 18 minutes e.g. at most 17 minutes. Typically, the onset of glucose lowering action (T_{GIRONSET}) following administration of 0.3 U/kg of the formulation is at least 13 minutes e.g. at least 15 minutes. An exemplary range is 13-21 minutes e.g. 13-20 minutes e.g. 15-19 minutes. The effect will for example be shown as a median effect in a group of at least 19 subjects.

In an embodiment, the time to onset of glucose lowering action (T_{GIRONSET}) following administration of 0.3 U/kg of the formulation is at most 90% than that of the corresponding value of Fiasp^{®}. Suitably, the time to onset of glucose lowering action (T_{GIRONSET}) is at most 85%, e.g. at most 80%, e.g.at most 75%, of that of the corresponding value of Fiasp^{®}. Typically, the time to onset of glucose lowering action (T_{GIRONSET}) following administration of 0.3 U/kg of the formulation is at least 60% e.g. at least 65% that of Fiasp^{®}. An exemplary range is 60-90% e.g. 60-85% e.g. 65-80% that of Fiasp^{®}. The effect will for example be shown as a median effect in a group of at least 19 subjects per treatment drug.

In an embodiment, the time to onset of glucose lowering action (T_{GIRONSET}) following administration of 0.3 U/kg of the formulation is at least 2 minutes less than that of the corresponding value of Fiasp^{®}. Suitably, the time to onset of glucose lowering action (T_{GIRONSET}) is at least 3 minutes, e.g. at least 4 minutes e.g. at least 5 minutes, e.g. at least 6 minutes less than that of the corresponding value of Fiasp^{®}. Typically, the time to onset of glucose lowering action (T_{GIRONSET}) following administration of 0.3 U/kg of the formulation is at most 10 minutes less e.g. at most 8 minutes less than that of Fiasp^{®} e.g. at most 8 minutes less than that of Fiasp^{®}. An exemplary range is 2-10 e.g. 4-10 e.g. 4-8 minutes less than that of Fiasp^{®}. The effect will for example be shown as a median effect in a group of at least 19 subjects per treatment drug.

In an embodiment, the time to 50% maximum glucose infusion rate in early (upward) part of the pharmacodynamic profile (T_{GIR50%MAX}) following administration of 0.3 U/kg of the formulation is at most 45 minutes. Suitably the time to 50% maximum glucose infusion rate in early (upward) part of the pharmacodynamic profile (T_{GIR50%MAX}) is at most 40 minutes e.g. at most 35 minutes e.g. at most 32 minutes e.g. at most 30 minutes. Typically, the time to 50% maximum glucose infusion rate in early (upward) part of the pharmacodynamic profile (T_{GIR50%MAX}) following administration of 0.3 U/kg of the formulation is at least 24 minutes e.g. at least 27 minutes. An exemplary range is 24-45 minutes e.g. 24-35 minutes e.g. 27-32 minutes. The effect will for example be shown as a median effect in a group of at least 19 subjects.

In an embodiment, the time to 50% maximum glucose infusion rate in early (upward) part of the pharmacodynamic profile (T_{GIR50%MAX}) following administration of 0.3 U/kg of the formulation is at most 90% of that of the corresponding value of Fiasp^{®}. Suitably the time to 50% maximum glucose infusion rate in early (upward) part of the pharmacodynamic profile (T_{GIR50%MAX}) is at most 80%, e.g. at most 70%, e.g. at most 65%, e.g. at most 60% of that of the corresponding value of Fiasp^{®}. Typically, the time to 50% maximum glucose infusion rate in early (upward) part of the pharmacodynamic profile (T_{GIR50%MAX}) following administration of 0.3 U/kg of the formulation is at least 50% e.g. at least 55% of that of the corresponding value of Fiasp^{®}. An exemplary range is 50-90% e.g. 50-70% e.g. 55-65% of that of the corresponding value of Fiasp^{®}. The effect will for example be shown as a median effect in a group of at least 19 subjects per treatment drug.

In an embodiment, the time to 50% maximum glucose infusion rate in early (upward) part of the pharmacodynamic profile (T_{GIR50%MAX}) following administration of 0.3 U/kg of the formulation is at least 5 minutes less than that of the corresponding value of Fiasp^{®}. Suitably the time to 50% maximum glucose infusion rate in early (upward) part of the pharmacodynamic profile (T_{GIR50%MAX}) is at least 8 minutes e.g. at least 12 minutes, e.g. at least 15 minutes e.g. at least 18 minutes e.g. at least 20 minutes less than that of that of the corresponding value of Fiasp^{®}. Typically, the time to 50% maximum glucose infusion rate in early (upward) part of the pharmacodynamic profile (T_{GIR50%MAX}) following administration of 0.3 U/kg of the formulation is at most 26 minutes less e.g. at most 23 minutes less than that of the corresponding value of Fiasp^{®}. An exemplary range is 5-26 minutes e.g. 15-26 minutes e.g. 18-23 minutes less that of the corresponding value of Fiasp^{®}. The effect will for example be shown as a median effect in a group of at least 19 subjects per treatment drug.

### Pharmacokinetic parameters

In an embodiment, the area under the baseline corrected insulin concentration curve t=0-30 minutes (AUC_{INSBC0-30}) following administration of 0.3 U/kg of the formulation is at least 24 mU×h/L, especially for insulin aspart. Suitably the area under the baseline corrected insulin concentration curve t=0-30 minutes (AUC_{INSBC0-30}) is at least 27 mU×h/L, e.g. at least 30 mU×h/L, e.g. at least 35 mU×h/L e.g. at least 38 mU×h/L, especially for insulin aspart. Typically, the area under the baseline corrected insulin concentration curve t=0-30 minutes (AUC_{INSBC0-30}) following administration of 0.3 U/kg of the formulation is at most 46 mU×h/L e.g. at most 43 mU×h/L, especially for insulin aspart. An exemplary range is 24-46 mU×h/L e.g. 30-46 mU×h/L e.g. 35-43 mU×h/L, especially for insulin aspart. The effect will for example be shown as a median effect in a group of at least 19 subjects.

In an embodiment, the area under the baseline corrected insulin concentration curve t=0-30 minutes (AUC_{INSBC0-30}) following administration of 0.3 U/kg of the formulation is at least 130% of that of the corresponding value of Fiasp^{®}, especially for insulin aspart. Suitably the area under the baseline corrected insulin concentration curve t=0-30 minutes (AUC_{INSBC0-30}) is at least 140%, e.g. at least 150%, e.g. at least 160%, e.g. at least 170%, e.g. at least 180% e.g. at least 190%, e.g. at least 200%, e.g. at least 210% of that of Fiasp^{®}, e.g. at least 220% of that of the corresponding value of Fiasp^{®}, especially for insulin aspart. Typically, the area under the baseline corrected insulin concentration curve t=0-30 minutes (AUC_{INSBC0-30}) following administration of 0.3 U/kg of the formulation is at most 250% e.g. at most 230% of that of the corresponding value of Fiasp^{®}, especially for insulin aspart. An exemplary range is 130-250% e.g. 170-250 % e.g. 190-230% of that of the corresponding value of Fiasp^{®}, especially for insulin aspart. The effect will for example be shown as a median effect or as a geometric mean effect in a group of at least 19 subjects per treatment drug.

In an embodiment, the area under the baseline corrected insulin concentration curve t=0-30 minutes (AUC_{INSBC0-30}) following administration of 0.3 U/kg of the formulation is at least 5 mU×h/L higher than that of the corresponding value of Fiasp^{®}, especially for insulin aspart. Suitably the area under the baseline corrected insulin concentration curve t=0-30 minutes (AUC_{INSBC0-30}) is at least 10 mU×h/L, e.g. at least 15 mU×h/L, e.g. at least 18 mU×h/L, e.g. is or is at least 20 mU×h/L higher than that of the corresponding value of Fiasp^{®}, especially for insulin aspart. Typically, the area under the baseline corrected insulin concentration curve t=0-30 minutes (AUC_{INSBC0-30}) following administration of 0.3 U/kg of the formulation is at most 28 mU×h/L e.g. at most 24 mU×h/L higher than that of that of the corresponding value of Fiasp^{®}, especially for insulin aspart. An exemplary range is 5-28 mU×h/L e.g. 15-28 mU×h/L e.g. 18-24 mU×h/L higher than that of the corresponding value of Fiasp^{®}, especially for insulin aspart. The effect will for example be shown as a median effect in a group of at least 19 subjects per treatment drug.

In an embodiment, the area under the baseline corrected insulin concentration curve t=0-60 minutes (AUC_{INSBC0-60}) following administration of 0.3 U/kg of the formulation is at least 80 mU×h/L, especially for insulin aspart. Suitably the area under the baseline corrected insulin concentration curve t=0-60 minutes (AUC_{INSBC0-60}) is at least 85 mU×h/L, e.g. at least 90 mU×h/L, e.g. at least 100 mU×h/L e.g. at least 110 mU×h/L, especially for insulin aspart. Typically, the area under the baseline corrected insulin concentration curve t=0-60 minutes (AUC_{INSBC0-60}) following administration of 0.3 U/kg of the formulation is at most 130 mU×h/L e.g. at most 120 mU×h/L, especially for insulin aspart. An exemplary range is 80-130 mU×h/L e.g. 90-130 mU×h/L e.g. 100-120 mU×h/L, especially for insulin aspart. The effect will for example be shown as a median effect in a group of at least 19 subjects.

In an embodiment, the area under the baseline corrected insulin concentration curve t=0-60 minutes (AUC_{INSBC0-60}) following administration of 0.3 U/kg of the formulation is at least 125% of that of the corresponding value of Fiasp^{®}, especially for insulin aspart. Suitably, the area under the baseline corrected insulin concentration curve t=0-60 minutes (AUC_{INSBC0-60}) is at least 135% e.g. at least 140%, e.g. at least 150%, e.g. at least 160%, e.g. at least 170% of that of the corresponding value of Fiasp^{®}, especially for insulin aspart. Typically, the area under the baseline corrected insulin concentration curve t=0-60 minutes (AUC_{INSBC0-60}) following administration of 0.3 U/kg of the formulation is at most 205% e.g. at most 185% of that of the corresponding value of Fiasp^{®}, especially for insulin aspart. An exemplary range is 125-205% e.g. 150-205% e.g. 150-185% of that of the corresponding value of Fiasp^{®}, especially for insulin aspart. The effect will for example be shown as a median effect or as a geometric mean effect in a group of at least 19 subjects per treatment drug.

In an embodiment, the area under the baseline corrected insulin concentration curve t=0-60 minutes (AUC_{INSBC0-60}) following administration of 0.3 U/kg of the formulation is at least 15 mU×h/L higher than that of the corresponding value of Fiasp^{®}, especially for insulin aspart. Suitably, the area under the baseline corrected insulin concentration curve t=0-60 minutes (AUC_{INSBC0-60}) is at least 20 mU×h/L, e.g. at least 30 mU×h/L, e.g. at least 40 mU×h/L, e.g. at least 45 mU×h/L higher than that of the corresponding value of Fiasp^{®}, especially for insulin aspart. Typically, the area under the baseline corrected insulin concentration curve t=0-60 minutes (AUC_{INSBC0-60}) following administration of 0.3 U/kg of the formulation is at most 66 mU×h/L e.g. at most 55 mU×h/L higher than that of the corresponding value of Fiasp^{®}, especially for insulin aspart. An exemplary range is 15-66 mU×h/L e.g. 22-66 mU×h/L e.g. 30-55 mU×h/L higher than that of the corresponding value of Fiasp^{®}, especially for insulin aspart. The effect will for example be shown as a median effect in a group of at least 19 subjects per treatment drug.

In an embodiment, the time to initial onset of insulin exposure (T_{INSONSET}) following administration of 0.3 U/kg of the formulation is at most 4 minutes, especially for insulin aspart. Suitably the time to initial onset of insulin exposure (T_{INSONSET}) is at most 3 minutes e.g. at most 2.5 minutes e.g. at most 2 minutes, especially for insulin aspart. Typically, the time to initial onset of insulin exposure (T_{INSONSET}) following administration of 0.3 U/kg of the formulation is at least 1 minute e.g. at least 1.5 minutes. An exemplary range is 1-4 minutes e.g. 1-3 minutes, e.g. 1.5-2.5 minutes especially for insulin aspart. The effect will for example be shown as a median effect in a group of at least 19 subjects.

In an embodiment, the time to initial onset of insulin exposure (T_{INSONSET}) following administration of 0.3 U/kg of the formulation is at most 40% of that of the corresponding value of Fiasp^{®}, especially for insulin aspart. Typically, the time to initial onset of insulin exposure (T_{INSONSET}) following administration of 0.3 U/kg of the formulation is at least 25% e.g. at least 30% of that of the corresponding value of Fiasp^{®}, especially for insulin aspart. An exemplary range is 30-50% of that of the corresponding value of Fiasp^{®}, especially for insulin aspart. The effect will for example be shown as a median effect in a group of at least 19 subjects per treatment drug.

In an embodiment, the time to initial onset of insulin exposure (T_{INSONSET}) following administration of 0.3 U/kg of the formulation is at least 1 minute less than that of the corresponding value of Fiasp^{®}, especially for insulin aspart. Suitably, the time to initial onset of insulin exposure (T_{INSONSET}) is at least 1.5 minutes, e.g. at least 2.0 minutes e.g. at least 2.5 minutes less than that of the corresponding value of Fiasp^{®}, especially for insulin aspart. Typically, the time to initial onset of insulin exposure (T_{INSONSET}) following administration of 0.3 U/kg of the formulation is at most 4 minutes less e.g. at most 3.5 minutes less than that of the corresponding value of Fiasp^{®}, especially for insulin aspart. An exemplary range is 1-4 minutes e.g. 1.5-4 minutes e.g. 2.5-3.5 minutes less than that of the corresponding value of Fiasp^{®}, especially for insulin aspart. The effect will for example be shown as a median effect in a group of at least 19 subjects per treatment drug.

In an embodiment, the time to maximum insulin concentration in the early (upward) part of the baseline corrected insulin curve (T_{INSMAX}) following administration of 0.3 U/kg of the formulation is at most 70 minutes, especially for insulin aspart. Suitably, the time to maximum insulin concentration in the early (upward) part of the baseline corrected insulin curve (T_{INSMAX}) is at most 65 minutes, e.g. at most 60 minutes, e.g. at most 55 minutes, e.g. at most 50 minutes, especially for insulin aspart. Typically, the time to maximum insulin concentration in the early (upward) part of the baseline corrected insulin curve (T_{INSMAX}) following administration of 0.3 U/kg of the formulation is at least 40 minutes e.g. at least 45 minutes, especially for insulin aspart. An exemplary range is 40-70 minutes e.g. 40-60 minutes e.g. 45-55 minutes, especially for insulin aspart. The effect will for example be shown as a median effect in a group of at least 19 subjects.

In an embodiment, the time to maximum insulin concentration in the early (upward) part of the baseline corrected insulin curve (T_{INSMAX}) following administration of 0.3 U/kg of the formulation is at most 90% of that of the corresponding value of Fiasp^{®}, especially for insulin aspart. Suitably, the time to maximum insulin concentration in the early (upward) part of the baseline corrected insulin curve (T_{INSMAX}) is at most 85%, e.g. at most 80%, e.g. at most 75%, e.g. at most 70% of that of the corresponding value of Fiasp^{®}, especially for insulin aspart. Typically, the time to maximum insulin concentration in the early (upward) part of the baseline corrected insulin curve (T_{INSMAX}) following administration of 0.3 U/kg of the formulation is at least 50% e.g. at least 60% of that of the corresponding value of Fiasp^{®}, especially for insulin aspart. An exemplary range is 50-90% e.g. 50-80% e.g. 60-75% of that of the corresponding value of Fiasp^{®}, especially for insulin aspart. The effect will for example be shown as a median effect in a group of at least 19 subjects per treatment drug.

In an embodiment, the time to maximum insulin concentration in the early (upward) part of the baseline corrected insulin curve (T_{INSMAX}) following administration of 0.3 U/kg of the formulation is at least 8 minutes less than that of the corresponding value of Fiasp^{®}, especially for insulin aspart. Suitably, the time to maximum insulin concentration in the early (upward) part of the baseline corrected insulin curve (T_{INSMAX}) is at least 12 minutes, e.g. at least 15 minutes, e.g. at least 18 minutes, e.g. at least 20 minutes, e.g. at least 22 minutes, e.g. at least 25 minutes less than that of the corresponding value of Fiasp^{®}, especially for insulin aspart. Typically, the time to maximum insulin concentration in the early (upward) part of the baseline corrected insulin curve (T_{INSMAX}) following administration of 0.3 U/kg of the formulation is at most 35 minutes less e.g. at most 30 minutes less than that of the corresponding value of Fiasp^{®}, especially for insulin aspart. An exemplary range is 8-35 minutes e.g. 15-35 minutes e.g. 20-30 minutes less than that of the corresponding value of Fiasp^{®}, especially for insulin aspart. The effect will for example be shown as a median effect in a group of at least 19 subjects per treatment drug.

In an embodiment, the time to 50% maximum insulin concentration in the early (upward) part of the baseline corrected insulin curve (T_{INS50%MAX}) following administration of 0.3 U/kg of the formulation is at most 20 minutes, especially for insulin aspart. Suitably, the time to 50% maximum insulin concentration in the early (upward) part of the baseline corrected insulin curve (T_{INS50%MAX}) is at most 18 minutes e.g. at most 16 minutes e.g. at most 14 minutes e.g. at most 12 minutes, especially for insulin aspart. Typically, the time to 50% maximum insulin concentration in the early (upward) part of the baseline corrected insulin curve (T_{INS50%MAX}) following administration of 0.3 U/kg of the formulation is at least 9 minutes, e.g. at least 10 minutes, especially for insulin aspart. An exemplary range is 9-20 minutes e.g, 9-16 minutes e.g 10-14 minutes, especially for insulin aspart. The effect will for example be shown as a median effect in a group of at least 19 subjects.

In an embodiment, the time to 50% maximum insulin concentration in the early (upward) part of the baseline corrected insulin curve (T_{INS50%MAX}) following administration of 0.3 U/kg of the formulation is at most 85% of that of the corresponding value of Fiasp^{®}, especially for insulin aspart. Suitably, the time to 50% maximum insulin concentration in the early (upward) part of the baseline corrected insulin curve (T_{INS50%MAX}) is at most 80% of that, e.g. at most 75%, e.g. at most 70%, e.g. at most 65%, e.g. at most 60%, e.g. at most 55%, e.g. at most 50% of that of the corresponding value of Fiasp^{®}, especially for insulin aspart. Typically, the time to 50% maximum insulin concentration in the early (upward) part of the baseline corrected insulin curve (T_{INS50%MAX}) following administration of 0.3 U/kg of the formulation is at least 40% e.g. at least 45% of that of the corresponding value of Fiasp^{®}, especially for insulin aspart. An exemplary range is 40-85% e.g. 40-60% e.g. 45-55% of that of the corresponding value of Fiasp^{®}, especially for insulin aspart. The effect will for example be shown as a median effect in a group of at least 19 subjects per treatment drug.

In an embodiment, the time to 50% maximum insulin concentration in the early (upward) part of the baseline corrected insulin curve (T_{INS50%MAX}) following administration of 0.3 U/kg of the formulation is at least 4 minutes less than that of that of the corresponding value of Fiasp^{®}, especially for insulin aspart. Suitably, the time to 50% maximum insulin concentration in the early (upward) part of the baseline corrected insulin curve (T_{INS50%MAX}) is at least 6 minutes, e.g. at least 8 minutes, e.g. at least 10 minutes, e.g. at least 12 minutes less than that of the corresponding value of Fiasp^{®}, especially for insulin aspart. Typically, the time to 50% maximum insulin concentration in the early (upward) part of the baseline corrected insulin curve (T_{INS50%MAX}) following administration of 0.3 U/kg of the formulation is at most 16 minutes less e.g. at most 14 minutes less that of the corresponding value of Fiasp^{®}, especially for insulin aspart. An exemplary range is 4-16 minutes e.g. 8-16 minutes e.g. 10-14 minutes less than that of the corresponding value of Fiasp^{®}, especially for insulin aspart. The effect will for example be shown as a median effect in a group of at least 19 subjects per treatment drug.

In an embodiment, the time to 50% maximum insulin concentration in the late (downward) part of the baseline corrected insulin curve (T_{INS50%MAXLATE}) following administration of 0.3 U/kg of the formulation is at most 205 minutes, especially for insulin aspart. Suitably, the time to 50% maximum insulin concentration in the late (downward) of the baseline corrected insulin curve (T_{INS50%MAXLATE}) is at most 200 minutes, e.g. at most 190 minutes, e.g. at most 180 minutes, e.g. at most 175 minutes, especially for insulin aspart. Typically, the time to 50% maximum insulin concentration in the late (downward) part of the baseline corrected insulin curve (T_{INS50%MAXLATE}) following administration of 0.3 U/kg of the formulation is at least 135 minutes e.g. at least 150 minutes, especially for insulin aspart. An exemplary range is 135-205 minutes e,g, 135-190 minutes e,g, 150-190 minutes e.g. 150-185 minutes, especially for insulin aspart. The effect will for example be shown as a median effect in a group of at least 19 subjects.

In an embodiment, the time to 50% maximum insulin concentration in the late (downward) part of the baseline corrected insulin curve (T_{INS50%MAXLATE}) following administration of 0.3 U/kg of the formulation is at most 93% of that of the corresponding value of Fiasp^{®}, especially for insulin aspart. Suitably, the time to 50% maximum insulin concentration in the late (downward) part of the baseline corrected insulin curve (T_{INS50%MAXLATE}) is at most 90% e.g. at most 88%, e.g. at most 85%, e.g. at most 80% of that of the corresponding value of Fiasp^{®}, especially for insulin aspart. Typically, the time to 50% maximum insulin concentration in the late (downward) part of the baseline corrected insulin curve (T_{INS50%MAXLATE}) following administration of 0.3 U/kg of the formulation is at least 60% e.g. at least 70% of that of the corresponding value of Fiasp^{®}, especially for insulin aspart. An exemplary range is 60-93% e.g. 60-88% e.g. 70-85% of that of the corresponding value of Fiasp^{®}, especially for insulin aspart. The effect will for example be shown as a median effect in a group of at least 19 subjects per treatment drug.

In an embodiment, the time to 50% maximum insulin concentration in the late (downward) part of the baseline corrected insulin curve (T_{INS50%MAXLATE}) following administration of 0.3 U/kg of the formulation is at least 16 minutes less than that of the corresponding value of Fiasp^{®}, especially for insulin aspart. Suitably, the time to 50% maximum insulin concentration in the late (downward) part of the baseline corrected insulin curve (T_{INS50%MAXLATE}) is at least 18 minutes, e.g. at least 20 minutes, e.g. at least 22 minutes, e.g. at least 25 minutes, e.g. at least 27 minutes, e.g. at least 30 minutes less than that of the corresponding value of Fiasp^{®}, especially for insulin aspart. Typically, the time to 50% maximum insulin concentration in the late (downward) part of the baseline corrected insulin curve (T_{INS50%MAXLATE}) following administration of 0.3 U/kg of the formulation is at most 80 minutes e.g. at most 75 minutes less e.g. at most 65 minutes less than that of the corresponding value of Fiasp^{®}, especially for insulin aspart. An exemplary range is 16-80 minutes e.g. 25-75 minutes less e.g. 27-65 minutes less than that of the corresponding value of Fiasp^{®}, especially for insulin aspart. The effect will for example be shown as a median effect in a group of at least 19 subjects per treatment drug.

As used above, the expression "the corresponding value of Fiasp^{®}" is the value obtained when Fiasp^{®} is administered in the same manner as the formulation of the invention with which it is being compared.

### Dose/administration

In one embodiment, the formulation is administered by subcutaneous injection. In another embodiment, the formulation is administered by subcutaneous infusion.

The formulation for use according to the invention is administered as a prandial bolus.

The bolus administration should occur in the window between 20 minutes after the start of the meal and 30 minutes after the start of the meal. A typical bolus dose of the fast acting insulin analogue in a formulation of the invention is 2-30 U, e.g. 5-15 U.

Subcutaneous injection may be by conventional syringe or more preferably via a pen device adapted for use by diabetic subjects. Exemplary pen devices include the Kwikpen^{®} device and the Flexpen^{®} device.

In one embodiment, the formulation for use according to the invention is administered continuously to provide a constant basal level of insulin, e.g. by administering a small pulse of the formulation every 5 minutes. A typical rate of administration of the formulation of the invention to provide a constant basal level of insulin is 0.2-2 U/h, such as 0.5-1.5 U/h.

Subcutaneous infusion may be by a medical device comprising a reservoir comprising a plurality of doses of the formulation and a pump adapted for automatic or remote operation such that upon automatic or remote operation one or more doses of the formulation. Such devices may be worn on the outside of the body or implanted in the body. Such devices are typically capable of delivering the formulation of the invention both as a bolus and at a specified basal rate. The device may be part of a system comprising an insulin sensor and a controller wherein the controller determines the rate of delivery of the formulation of the invention based on the signal from the glucose sensor.

In one embodiment, the formulation for use according to the invention is co-administered with another agent of use in the treatment of diabetes (i.e. in addition to the fast acting insulin analogue) e.g. an amylin analogue or a GLP-1 agonist. In one embodiment, the formulation of the invention is co-administered with an amylin analogue such as pramlintide, suitably at a concentration of 0.1-10 mg/ml e.g. 0.2-6 mg/ml. In one embodiment, the formulation for use according to the invention is co-administered with a GLP-1 agonist such as liraglutide, dulaglutide, albiglutide, exenatide, semaglutide or lixisenatide, suitably at a concentration of 10 µg/ml to 50 mg/ml e.g. 200 µg/ml to 10 mg/ml or 1-10 mg/ml.

In one embodiment, the formulation for use according to the invention is co-administered with a long acting insulin such as insulin glargine or insulin degludec, suitably at a concentration of 50-1000 U/ml e.g. 100-500 U/ml or 100-200 U/ml.

Formulations may be prepared by mixing the ingredients. For example, the fast acting insulin analogue may be dissolved in an aqueous formulation comprising the other components. Alternatively, the fast acting insulin analogue may be dissolved in a strong acid (typically HCl), after dissolution diluted with an aqueous formulation comprising the other components, and then pH adjusted to the desired pH with addition of alkali (e.g. NaOH). As a variation on this method, a step of neutralising the acid solution may be performed before the dilution step and it may then not be necessary to adjust the pH after the dilution step (or a small adjustment only may be necessary).

Methods and uses in at least some embodiments are expected to have one or more of the following advantageous properties:
- Ultra rapid action for example demonstrated by one or more of the pharmacodynamic parameters AUC_{GIR0-30}, AUC_{GIR0-60}, T_{GIRONSET} and T_{GIR50%MAX} and the pharmacokinetic parameters AUC_{INSBC0-30}, AUC_{INSBC0-60}, T_{INSONSET}, T_{INSMAX}, T_{INS50%MAX} and T_{INS50%MAXLATE}.
- Good physical and chemical stability.

### ABBREVIATIONS

- EDTA: ethylenediaminetetraacetate
- EGTA: ethyleneglycoltetraacetate
- HPLC: high performance liquid chromatography
- HMWS: high molecular weight species
- RP: reverse phase
- SEC: size-exclusion chromatography
- PD: pharmacodynamic
- PK: pharmacokinetic

### EXAMPLES

### General methods

### (a) The Diabetic Pig Pharmacokinetic/Pharmacodynamic Model: Method for determining speed of action:

10 male diabetic Yucatan miniature pigs may be used. Pigs are injected subcutaneously with a sample of the test formulation and blood is taken (1 or 2 ml) at various time-points (min) with respect to the injection up to around 240 min after the injection. For pharmacodynamics profile, serum is analysed for glucose (using a commercially available glucometer). For pharmacokinetic profile, insulin concentration is determined in the serum using an immunoassay.

In order to evaluate relative speed of action of formulations, mean values of T_{MAX} (i.e. time to reach the maximum insulin concentration in serum) are calculated across the whole set of 10 pigs used in the study. Insulin analogues with a T_{MAX} which is less than insulin are fast acting insulin analogues.

### Example 1 - Example formulations

The following example formulations may be prepared:

### Example A:

| | |
|---|---|
| Insulin lispro | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 22 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example B:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 22 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example C:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 22 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example D:

| | |
|---|---|
| Insulin glulisine | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 22 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example E:

| | |
|---|---|
| Insulin lispro | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 22 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

### Example F:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 22 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example G:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 22 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

### Example H

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 22 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH was adjusted to 7.4 | |

### Example I:

| | |
|---|---|
| Insulin aspart | 200 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 22 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example J:

| | |
|---|---|
| Insulin aspart | 200 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 22 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example K:

| | |
|---|---|
| Insulin lispro | 200 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 22 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

### Example L:

| | |
|---|---|
| Insulin aspart | 200 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 22 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

### Example M:

| | |
|---|---|
| Insulin lispro | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 44 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example N:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 44 mM |
| Glycerol | 174 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example O:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 44 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl p |
| H adjusted to 7.4 | |

### Example P:

| | |
|---|---|
| Insulin aspart | 100 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citric acid | 44 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

### Example Q:

| | |
|---|---|
| Insulin aspart | 200 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 44 mM |
| Glycerol | 174 mM |
| Surfactant | dodecyl maltoside (0.05 mg/ml) |
| Water for injection | qs |
| Residual NaCl | Acidification and subsequent neutralisation during preparationresults in formation of 2-4 mM NaCl |
| pH adjusted to 7.4 | |

### Example R:

| | |
|---|---|
| Insulin aspart | 200 U/ml |
| Sodium phosphate | 2 mM |
| phenol | 15.9 mM |
| m-cresol | 15.9 mM |
| Ionic zinc (as ZnCl₂) | 19.7 µg/ml (0.3 mM), equals 0.55% (w/w) based on the weight of fast acting insulin analogue in the formulation |
| Citrate (as trisodium salt) | 44 mM |
| NaCl | 150 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Method for preparation for the above formulations:
Insulin powder is added to water and HCl is added until the powder is fully dissolved (pH has to be <3 in order to achieve full dissolution). ZnCl₂ is added to the required level. Once dissolved, pH is adjusted to approximately 7 and volume is adjusted with water so that the insulin concentration is 2× the required concentration. The composition is then mixed 1:1 (v/v) with a mixture of additional excipients (all at 2× the required concentration).

### Example 2 - Phase I glucose clamp study comparing fast acting insulin aspart formulation of the invention and commercial fast acting insulin aspart products

A phase I, single dose, randomised, double-blind, three-way cross over, glucose clamp study investigating the pharmacodynamics and pharmacokinetics of an insulin aspart formulation comprising ionic zinc and citrate (AT500) in comparison to commercial insulin aspart formulations NovoRapid^{®} and Fiasp^{®} (all "fast acting insulin formulations") in participants with type 1 diabetes mellitus was carried out.

### Methods

A randomised, double-blind, single-dose, single-centre, three-period, complete crossover phase 1 trial including 19 male participants with type I diabetes was carried out. At each dosing visit the participants underwent an 8 hour euglycaemic clamp procedure. During the run-in period before the clamp procedure, participants received a variable intravenous infusion of human insulin or glucose to obtain a blood glucose target level of 100 mg/dL (5.5 mmol/L). Once the blood glucose had been stable for at least 1 hour without glucose infusion, a fast acting insulin formulation was administered by subcutaneous injection at a dose of 0.3 U/kg. The administration of the fast acting insulin formulation is referred to as time-point 0. Fast acting insulin formulation administration at time-point 0 marked the end of the run-in period and the start of the clamp period.

When plasma glucose dropped by 5 mg/dL relative to the baseline level, i.e. to 95 mg/dL (5.3 mmol/L), a variable intravenous glucose infusion was initiated to keep the plasma glucose concentration constant at the clamp target 100 mg/dL (5.5 mmol/L). Baseline was defined as the mean glucose concentration between -10 minutes to 0 minutes of fast acting insulin formulation administration. The rate of fast acting insulin formulation infusion required to maintain glucose concentration at the baseline level is referred to as 'glucose infusion rate'. The euglycaemic clamp lasted for up to 8 hours after trial product administration. The euglycaemic clamp lasted for up to 8 hours after fast acting insulin formulation administration. Regular blood samples were taken throughout the duration of the clamp. Plasma glucose concentration was measured using Super GL 2 Glucose analyser. Serum concentration of fast acting insulin analogue (e.g. insulin aspart) was measured by ELISA (Mercordia Iso-Insulin ELISA). Human insulin, administered during the run-in period, was measured by the human insulin selective ELISA (Mercordia insulin ELISA) at time points up to 40 minutes. The fast acting Insulin analogue ELISA results were corrected by subtraction of the human insulin ELISA results. The resulting results are referred to as Baseline corrected insulin concentration (as mentioned below, "insulin" in this context means the fast acting insulin analogue administered, e.g. insulin aspart).

The pharmacodynamics and pharmacokinetics of a composition according to the present claims comprising (i) a fast acting insulin analogue; (ii) ionic zinc; and (iii) citrate, said formulation being substantially free of a zinc binding species having a logK with respect to zinc ion binding of greater than 12.3 at 25 °C (AT500) was compared with that of commercial insulin aspart products, NovoRapid^{®} and Fiasp^{®}.

The composition of the fast acting insulin formulation AT500, an embodiment of the present invention, of which test results are reported herein is as follows:
- Insulin aspart (100 U/ml)
- Sodium phosphate
- Citric acid
- Sodium chloride
- Dodecyl maltoside
- m-Cresol
- Phenol
- Water for injection qs
- pH was adjusted to 7.4 using sodium hydroxide

The composition of commercially available NovoRapid^{®} is as follows:
- Insulin aspart (100 U/ml)
- Sodium phosphate (8.8 mM)
- Sodium chloride (9.9 mM)
- Glycerol (174 mM)
- m-Cresol (15.9 mM)
- Phenol (15.9 mM)
- Water for injection qs
- pH was adjusted to 7.4 using sodium hydroxide

The composition of commercially available Fiasp^{®} is as follows:
- Insulin aspart (100 U/ml)
- Sodium phosphate (8.8 mM)
- Sodium chloride (9.9 mM)
- Glycerol (174 mM)
- m-Cresol (15.9 mM)
- Phenol (15.9 mM)
- Arginine hydrochloride (20 mM)
- Nicotinamide (vitamin B₃) (170 mM)
- pH was adjusted to 7.1 using sodium hydroxide

The compositions AT500, NovoRapid^{®} and Fiasp^{®} all contain ionic zinc.

The pharmacodynamic parameters measured in the study included:
- Area under the glucose infusion rate curve t=0-30 min (**AUC_{GIR0-30}**); i.e. the area under the glucose infusion rate curve between time-point 0 and 30 min.
- Area under the glucose infusion rate curve t=0-60 min (**AUC_{GIR0-60}**); i.e. the area under the glucose infusion rate curve between time-point 0 and 60 min.
- Time to onset of glucose lowering action (**T_{GIRONSET}**); i.e. time from time-point 0 to the first measurable increase in the glucose infusion rate.
- Time to 50% maximum glucose infusion rate in early (upward) part of the pharmacodynamic profile (**T_{GIR50%MAX}**); i.e. time from time-point 0 to the first time-point on the glucose infusion rate curve that exceeds 50% of the maximum glucose infusion rate.

The pharmacokinetic parameters measured in the study included:
- Area under the baseline corrected insulin concentration curve t=0-30 mins (**AUC_{INSBC0-30}**); i.e. the area under the baseline corrected insulin concentration curve between time-point 0 and 60 min.
- Area under the baseline corrected insulin concentration curve t=0-60 mins (**AUC_{INSBC0-60}**); i.e. the area under the baseline corrected insulin concentration curve between time-point 0 and 60 min.
- Time to initial onset of insulin exposure (**T_{INSONSET}**); i.e. time from time-point 0 to the first measurable increase in the baseline corrected insulin concentration.
- Time to maximum insulin concentration in the early (upward) part of the baseline corrected insulin curve (**T_{INSMAX}**); i.e. time from time-point 0 to the time of the maximum value on the baseline corrected insulin curve.
- Time to 50% maximum insulin concentration in early (upward) part of the baseline corrected insulin curve (**T_{INS50%MAX}**); i.e. time from time-point 0 to the first time-point that exceeds 50% of the maximum value on the baseline corrected insulin curve.
- Time to 50% maximum insulin concentration in the late (downward) part of the baseline corrected insulin curve (**T_{INS50%MAXLATE}**); i.e. time from time-point 0 to the last time-point that exceeds 50% of the maximum value on the baseline corrected insulin curve.

For all parameters, the median value, the geometric mean value and the geometric standard deviation were assessed.

As used herein, when "insulin" is referred to in connection with pharmacokinetic parameters, it refers to the form of insulin administered (i.e. the fast acting insulin analogue administered). Thus, in the study described in these Examples, it is insulin aspart.

### Pharmacodynamic results

### Mean Glucose Infusion Rate profile

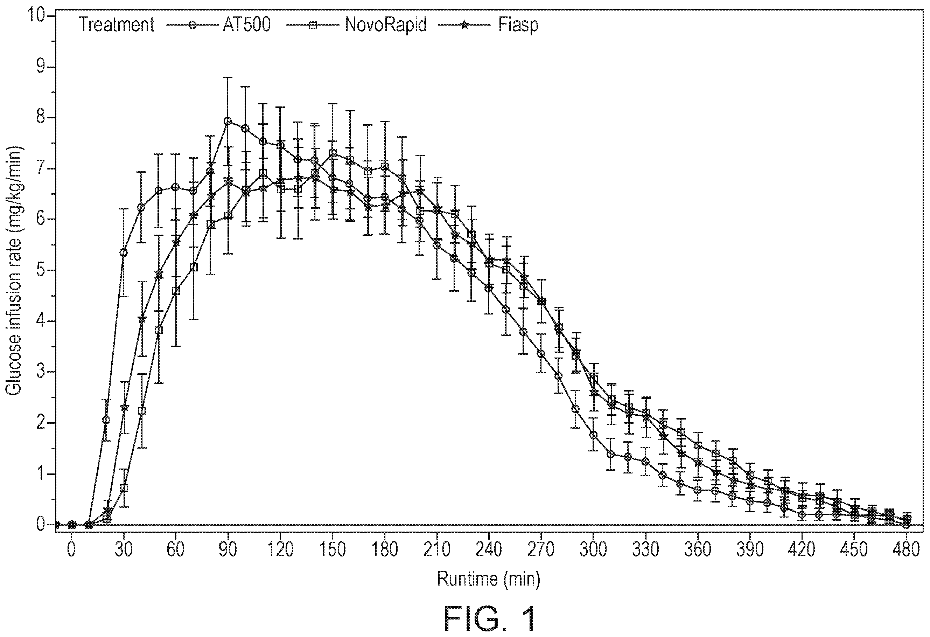

The mean Glucose Infusion Rate (GIR) profile of AT500 is shown in Figure 1, where it can be seen that AT500 showed a faster onset and greater early glucose-lowering effect compared with both Fiasp^{®} and NovoRapid^{®}.

The median and geometric mean values pharmacodynamic parameters of the three fast acting insulin formulations are shown in Table 1.

**Table 1: Median values of pharmacodynamic parameters measured in the study for AT500, Fiasp^{®} and NovoRapid^{®}.**

| **Parameter** | **Product** | **Median** | **AT500 Median / Fiasp^{®} Median** |
|---|---|---|---|
| AUC_{GIR0-30} (mg/kg) | NovoRapid^{®} | 0.00 | |
| AUC_{GIR0-30} (mg/kg) | Fiasp^{®} | 9.30 | |
| AUC_{GIR0-30} (mg/kg) | AT500 | 39.65 | 4.3 |
| AUC_{GIR0-60} (mg/kg) | NovoRapid^{®} | 48.55 | |
| AUC_{GIR0-60} (mg/kg) | Fiasp^{®} | 90.95 | |
| AUC_{GIR0-60} (mg/kg) | AT500 | 211.82 | 2.3 |
| T_{GIRONSET} (min) | NovoRapid^{®} | 37.00 | |
| T_{GIRONSET} (min) | Fiasp^{®} | 23.00 | |
| T_{GIRONSET} (min) | AT500 | 17.00 | 0.74 |
| T_{GIR50%MAX} (min) | NovoRapid^{®} | 65.00 | |
| T_{GIR50%MAX} (min) | Fiasp^{®} | 50.00 | |
| T_{GIR50%MAX} (min) | AT500 | 30.00 | 0.60 |

Treatment comparisons between AT500 and Fiasp^{®} and AT500 and NovoRapid^{®} were performed using the Koch's adaptation of Wilcoxon's rank sum test (as described in Senn S., Cross-over Trials in Clinical Research, Statistics in Practice, 2^{nd} Edition, John Willey & Sons Ltd. (2002), Chapter 4.3.9.). The median difference in the pharmacodynamic parameters and the p-values assessed by the Wilcoxon's rank sum test are shown in Table 2.

**Table 2: Treatment comparisons between AT500 and Fiasp^{®} and AT500 and NovoRapid^{®}. Median difference and p-value, as assessed by Koch's adaptation of Wilcoxon's rank sum test, is shown.**

| **Parameter** | **Product** | **Median difference** | **p-value** |
|---|---|---|---|
| AUC_{GIR0-30} (mg/kg) | AT500-NovoRapid^{®} | 36.79 | 0.0004 |
| AUC_{GIR0-30} (mg/kg) | AT500-Fiasp^{®} | 18.94 | 0.0007 |
| AUC_{GIR0-60} (mg/kg) | AT500-NovoRapid^{®} | 143.86 | 0.0009 |
| AUC_{GIR0-60} (mg/kg) | AT500-Fiasp^{®} | 81.43 | 0.0004 |
| T_{GIRONSET} (min) | AT500-NovoRapid^{®} | -23.00 | 0.0004 |
| T_{GIRONSET} (min) | AT500-Fiasp^{®} | -9.00 | 0.0006 |
| T_{GIR50%MAX} (min) | AT500-NovoRapid^{®} | -32.50 | 0.0004 |
| T_{GIR50%MAX} (min) | AT500-Fiasp^{®} | -20.00 | 0.0155 |

The above table shows that the differences between the formulation of the invention and the NovoRapid^{®} and Fiasp^{®} formulations are statistically significant (p< 0.02 and p<0.001 in most cases)

### Pharmacokinetic results

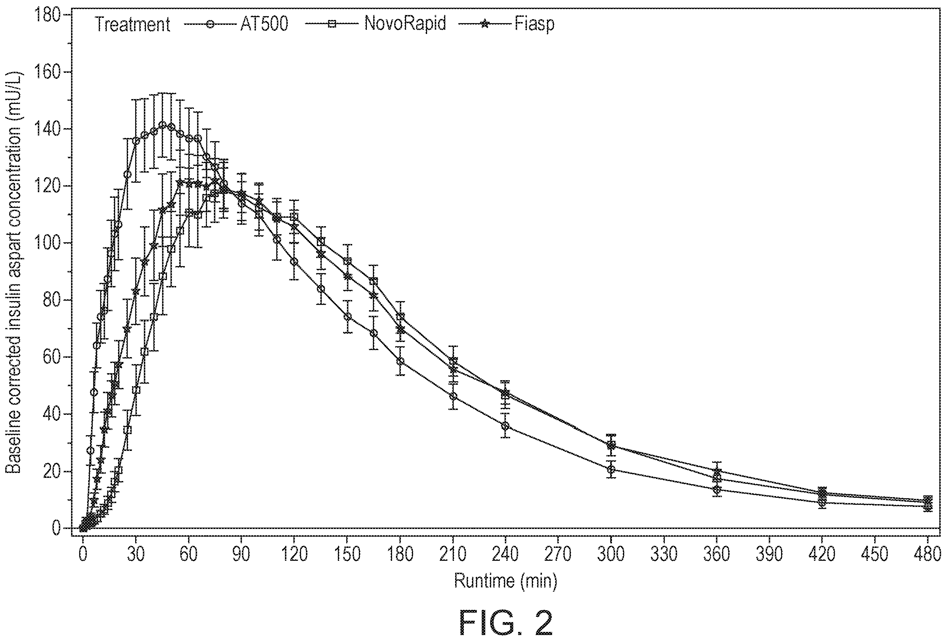

As can be seen from Figure 2, the pharmacokinetic profile also showed a faster onset and greater early insulin exposure with AT500 compared with both Fiasp^{®} and NovoRapid^{®}.

The median values of the main pharmacokinetic parameters of the three insulin products are shown in Table 3.

**Table 3: Median values of pharmacokinetic parameters measured in the study for AT500, Fiasp^{®} and NovoRapid^{®}.**

| **Parameter** | **Product** | **Median** | **AT500 Median / Fiasp^{®} Median** |
|---|---|---|---|
| AUC_{INSBC0-30} (mU×h/L) | NovoRapid^{®} | 7.13 | |
| AUC_{INSBC0-30} (mU×h/L) | Fiasp^{®} | 18.76 | |
| AUC_{INSBC0-30} (mU×h/L) | AT500 | 38.52 | 2.1 |
| AUC_{INSBC0-60} (mU×h/L) | NovoRapid^{®} | 41.40 | |
| AUC_{INSBC0-60} (mU×h/L) | Fiasp^{®} | 66.22 | |
| AUC_{INSBC0-60} (mU×h/L) | AT500 | 110.62 | 1.7 |
| T_{INSONSET} (min) | NovoRapid^{®} | 13.50 | |
| T_{INSONSET} (min) | Fiasp^{®} | 5.00 | |
| T_{INSONSET} (min) | AT500 | 2.00 | 0.40 |
| T_{INSMAX} (min) | NovoRapid^{®} | 90.00 | |
| T_{INSMAX} (min) | Fiasp^{®} | 75.00 | |
| T_{INSMAX} (min) | AT500 | 50.00 | 0.67 |
| T_{INS50%MAX} (min) | NovoRapid^{®} | 37.50 | |
| T_{INS50%MAX} (min) | Fiasp^{®} | 24.00 | |
| T_{INS50%MAX} (min) | AT500 | 12.00 | 0.50 |
| T_{INS50%MAXLATE} (min) | NovoRapid^{®} | 211.50 | |
| T_{INS50%MAXLATE} (min) | Fiasp^{®} | 221.00 | |
| T_{INS50%MAXLATE} (min) | AT500 | 173.00 | 0.78 |

Treatment comparisons between AT500 and Fiasp^{®} and AT500 and NovoRapid^{®} were performed using the Koch's adaptation of Wilcoxon's rank sum test (as described in Senn, *2002, infra)* except for the AUC parameters for which treatment comparisons between AT500 and Fiasp^{®} and AT500 and NovoRapid^{®} were performed using the Student's t test. The geometric mean of the AUC ratios and the p-values assessed by the Student's t test are shown in Table 4. The median difference in the pharmacokinetic parameters and the p-values assessed by the Wilcoxon's rank sum test are shown in Table 5.

**Table 4: Treatment comparisons between AT500 and Fiasp^{®} and AT500 and NovoRapid^{®}. Geometric mean of the AUC ratios and p value of the Student's t test**

| **Parameter** | **Product** | **Geometric mean of the AUC ratios** | **p-value** |
|---|---|---|---|
| AUC_{INSBC0-30} (mU×h/L) | AT500-NovoRapid^{®} | 6.91 | <0.0001 |
| AUC_{INSBC0-30} (mU×h/L) | AT500-Fiasp^{®} | 2.25 | <0.0001 |
| AUC_{INSBC0-60} (mU×h/L) | AT500-NovoRapid^{®} | 2.52 | <0.0001 |
| AUC_{INSBC0-60} (mU×h/L) | AT500-Fiasp^{®} | 1.56 | <0.0001 |

The above table shows that the differences between the formulation of the invention and the NovoRapid^{®} and Fiasp^{®} formulations are statistically significant (p<0.0001)

**Table 5: Treatment comparisons between AT500 and Fiasp^{®} and AT500 and NovoRapid^{®}. Median difference and p-value, as assessed by Koch's adaptation of Wilcoxon's rank sum test, is shown.**

| **Parameter** | **Product** | **Median difference** | **p-value** |
|---|---|---|---|
| T_{INSONSET} (min) | AT500-NovoRapid^{®} | -11.50 | 0.0004 |
| T_{INSONSET} (min) | AT500-Fiasp^{®} | -2.00 | 0.0003 |
| T_{INSMAX} (min) | AT500-NovoRapid^{®} | -35.00 | 0.0004 |
| T_{INSMAX} (min) | AT500-Fiasp^{®} | -25.00 | 0.0032 |
| T_{INS50%MAX} (min) | AT500-NovoRapid^{®} | -23.50 | 0.0004 |
| T_{INS50%MAX} (min) | AT500-Fiasp^{®} | -12.00 | 0.0004 |
| T_{INS50%MAXLATE} (min) | AT500-NovoRapid^{®} | -32.00 | 0.0015 |
| T_{INS50%MAXLATE} (min) | AT500-Fiasp^{®} | -27.00 | 0.0017 |

The above table shows that the differences between the formulation of the invention and the NovoRapid^{®} and Fiasp^{®} formulations are statistically significant (p< 0.005 and p<0.001 in most cases).

### Example 3 - Stability of insulin aspart formulation comprising ionic zinc and citrate (AT500)

Stability of the AT500 formulation was assessed at 2-8°C and 25°C using SEC, RP-HPLC and visual assessment. The following impurities were quantified by RP-HPLC method, in line with the US Pharmacopeia (USP38) monograph (Insulin Aspart Injection): B28isoAsp, A21Asp, B3Asp, B3isoAsp and total other related species. The SEC method was used to quantify high molecular weight species.

### General Methods for stability testing

### Size exclusion chromatography (SEC)

Ultra-high performance size exclusion chromatography of insulin preparations was performed using the Waters ACQUITY H-class Bio UPLC^{®} system with a 1.7 µm Ethylene Bridged Hybrid 125 Å pore packing material in a 300 mm by 4.6 mm column. The column was equilibrated in 0.65 mg/ml L-arginine, 20% v/v acetonitrile, 15%v/v glacial acetic acid mobile phase and 10 µl of sample, acidified with 0.01M HCl, was analysed at 0.4 mL/min, with 276 nm UV detection. All analyses were performed at ambient temperature.

### Reversed-phase chromatography (RP-HPLC)

Ultra-high performance reverse phase chromatography was performed using the Waters ACQUITY H-class Bio UPLC^{®} system with a 1.7 µm Ethylene Bridged Hybrid particle, 130 Å pore resin trifunctionally immobilised with a C18 ligand in a 50 mm by 2.1 mm column. Insulin samples were bound in a 82%w/v Na₂SO₄, 18% v/v acetonitrile, pH 2.3 mobile phase and eluted in 50% w/v Na₂SO₄, 50% v/v acetonitrile gradient flow. 2 µl of sample was acidified with 0.01M HCl and analysed at 0.61 mL/min, with 214 nm UV detection. All analyses were performed at 40°C.

### Visual assessment

Visible particles are suitably detected using the 2.9.20. European Pharmacopoeia Monograph (Particulate Contamination: Visible Particles). The apparatus required consists of a viewing station comprising:
- a matt black panel of appropriate size held in a vertical position
- a non-glare white panel of appropriate size held in a vertical position next to the black panel
- an adjustable lampholder fitted with a suitable, shaded, white-light source and with a suitable light diffuser (a viewing illuminator containing two 13 W fluorescent tubes, each 525 mm in length, is suitable). The intensity of illumination at the viewing point is maintained between 2000 lux and 3750 lux.

Any adherent labels are removed from the container and the outside washed and dried. The container is gently swirled or inverted, ensuring that air bubbles are not introduced, and observed for about 5 s in front of the white panel. The procedure is repeated in front of the black panel. The presence of any particles is recorded.

The visual scores are ranked as follows:

### Visual Assessment Scoring Method

Visual score 1: Clear solution, virtually free of particles
Visual score 2: ~ 5 very small particles
Visual score 3: ~10-20 very small particles
Visual score 4: 20-50 particles, including larger particles
Visual score 5: >50 particles, including larger particles

Whilst the particles in samples with visual scores 4 and 5 are clearly detectable on casual visual assessment under normal light, samples with visual score 1-3 generally appear as clear solutions on the same assessment. Samples with visual scores 1-3 are considered to be "Pass"; samples with visual score 4-5 are considered to be "Fail".

### Stability testing results

The results of the stability testing at 2-8°C are shown in Table 6. All parameters were within the specification following storage at 2-8°C for 12 months. The results of the stability testing at 25°C are shown in Table 7. All parameters were within the specification following storage at 25°C for 3 months. The level of two of the impurities (B28isoAsp and total other related species) was just outside the specified limit following storage at 25°C for 6 months. Other parameters were still within specification at this point.

**Table 6: Stability of AT500 formulation of insulin aspart at 2-8°C.**

| **Parameter** | **Specification** | **Initial** | **1 month** | **2 months** | **3 months** | **6 months** | **9 months** | **12 months** |
|---|---|---|---|---|---|---|---|---|
| Appearance | Clear and colourless | Pass | Pass | Pass | Pass | Pass | Pass | Pass |
| Visible Particles | Essentially free from particles | Pass | Pass | Pass | Pass | Pass | Pass | Pass |
| B28isoAsp | ≤ 2.5% | 0.19% | 0.16% | 0.31% | 0.29% | 0.37% | 0.428% | 0.60% |
| Total of all peaks due to A21Asp, B3Asp and B3isoAsp | ≤ 5% | 0.35% | 0.28% | 0.38% | 0.34% | 0.39% | 0.456% | 0.50% |
| Total other related species | ≤ 3.5% | 0.58% | 0.53% | 1.05% | 1.08% | 1.18% | 1.23% | 1.30% |
| HMWS | ≤ 1.5% | 0.12% | 0.13% | 0.18% | 0.19% | 0.20% | 0.25% | 0.23% |

**Table 7: Stability of AT500 formulation of insulin aspart at 25°C.**

| **Parameter** | **Specification** | **Initial** | **1 month** | **2 months** | **3 months** | **6 months** |
|---|---|---|---|---|---|---|
| Appearance | Clear and colourless | Pass | Pass | Pass | Pass | Pass |
| Visible Particles | Essentially free from particles | Pass | Pass | Pass | Pass | Pass |
| B28isoAsp | ≤ 2.5% | 0.19% | 0.72% | 1.60% | 2.01% | 3.53% |
| Total of all peaks due to A21Asp, B3Asp and B3isoAsp | ≤ 5% | 0.35% | 0.56% | 0.88% | 0.98% | 1.57% |
| Total other related species | ≤ 3.5% | 0.58% | 0.88% | 2.12% | 2.62% | 3.85% |
| HMWS | ≤ 1.5% | 0.12% | 0.18% | 0.35% | 0.39% | 0.76% |

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The term "and/or" as used in a phrase such as "A and/or B" herein is intended to include both A and B; A or B; A (alone); and B (alone). Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

### SEQUENCE LISTING

SEQ ID NO: 1: GIVEQCCTSICSLYQLENYCN
SEQ ID NO: 2: FVNQHLCGSHLVEALYLVCGERGFFYTPKT
SEQ ID NO: 3: FVNQHLCGSHLVEALYLVCGERGFFYTKPT
SEQ ID NO: 4: FVNQHLCGSHLVEALYLVCGERGFFYTDKT
SEQ ID NO: 5: FVKQHLCGSHLVEALYLVCGERGFFYTPET

## Claims

1. An aqueous liquid pharmaceutical formulation comprising (i) a fast acting insulin analogue; (ii) ionic zinc; and (iii) citrate, said formulation being substantially free of a zinc binding species having a logK with respect to zinc ion binding of greater than 12.3 at 25 °C; for use in the treatment of a human subject suffering from diabetes mellitus by administration by subcutaneous injection or subcutaneous infusion as a prandial bolus in the window between 20 minutes after the start of a meal and 30 minutes after the start of a meal,
wherein the administration of 0.3 U/kg of the formulation leads to:
a time to initial onset of insulin exposure (T_{INSONSET}) of at most 50%;
of the corresponding value upon administration of a formulation consisting of (i) insulin aspart at a concentration of 100 U/mL; (ii) sodium phosphate at a concentration of 8.8 mM;
(iii) sodium chloride at a concentration of 9.9 mM; (iv) glycerol at a concentration of 174 mM;
(v) m-cresol at a concentration of 15.9 mM; (vi) phenol at a concentration of 15.9 mM; (vii) arginine hydrochloride at a concentration of 20 mM; (viii) nicotinamide at a concentration of 170 mM; (ix) ionic zinc and (x) water; and with pH adjusted to 7.1 using sodium hydroxide.

2. The formulation for use according to claim 1, wherein the fast acting insulin analogue is insulin aspart.

3. The formulation for use according to claim 1, wherein the fast acting insulin analogue is insulin lispro.

4. The formulation for use according to any one of claims 1 to 3, wherein the fast acting insulin analogue is present at a concentration of 50-200 U/ml.

5. The formulation for use according to any one of claims 4 wherein the fast acting insulin analogue is present at a concentration of about 100 U/ml.

6. The formulation for use according to any one of claims 1 to 5, wherein the ionic zinc is present at a concentration of 0.5-1% by weight of zinc based on the weight of fast acting insulin analogue in the formulation.

7. The formulation for use according to any one of claims 1 to 6, wherein the source of the citrate is citric acid.

8. The formulation for use according to any one of claims 1 to 7, wherein citrate is present at a concentration of 1-50 mM.

9. The formulation for use according to any one of claims 1 to 8, wherein the molar ratio of ionic zinc to citrate is 1:3 to 1:175.

10. The formulation for use according to any one of claims 1 to 9, which is substantially free of zinc binding species having a logK with respect to zinc ion binding of 10-12.3 at 25 °C.

11. The formulation for use according to any one of claims 1 to 10, wherein the formulation further comprises a non-ionic surfactant, wherein the non-ionic surfactant is an alkyl glycoside selected from the group consisting of dodecyl maltoside, dodecyl glucoside, octyl glucoside, octyl maltoside, decyl glucoside, decyl maltoside, decyl glucopyranoside, tridecyl glucoside, tridecyl maltoside, tetradecyl glucoside, tetradecyl maltoside, hexadecyl glucoside, hexadecyl maltoside, sucrose monooctanoate, sucrose monodecanoate, sucrose monododecanoate, sucrose monotridecanoate, sucrose monotetradecanoate and sucrose monohexadecanoate; or
wherein the non-ionic surfactant is a polysorbate surfactant; or
wherein the non-ionic surfactant is an alkyl ether of polyethylene glycol selected from polyethylene glycol (2) dodecyl ether, polyethylene glycol (2) oleyl ether and polyethylene glycol (2) hexadecyl ether; or
wherein the non-ionic surfactant is a block copolymer of polyethylene glycol and polypropylene glycol selected from is poloxamer 188, poloxamer 407, poloxamer 171 or poloxamer 185; or
wherein the non-ionic surfactant is an alkylphenyl ether of polyethylene glycol;
wherein the non-ionic surfactant is present at a concentration of 1-1000 µg/ml.

12. The formulation for use according to claim 11, wherein the non-ionic surfactant is an alkyl glycoside selected from the group consisting of dodecyl maltoside and decyl glucopyranoside.

13. The formulation for use according to claim 12, wherein the alkyl glycoside is dodecyl maltoside.

14. The formulation for use according to any one of claims 1 to 13, further comprising an uncharged tonicity modifying agent, wherein the uncharged tonicity modifying agent is selected from the group consisting of trehalose, mannitol, glycerol and 1,2-propanediol.

15. The formulation for use according to any one of claims 1 to 13, further comprising a charged tonicity modifying agent present at a concentration of >60 mM wherein the charged tonicity modifying agent is sodium chloride.

16. The formulation for use according to any one of claims 1 to 15, wherein the pH is in the range 7.0 to 7.8.

17. The formulation for use according to any one of claims 1 to 16, further comprising nicotinamide; and/or
further comprising nicotinic acid or a salt thereof; and/or
further comprising treprostinil or a salt thereof; or
wherein the formulation does not contain a vasodilator.

18. The formulation for use according to any one of claims 1 to 17, wherein the formulation further comprises an additional therapeutically active agent of use in the treatment of diabetes selected from the group consisting of an amylin analogue and a GLP-1 agonist; and/or
wherein the formulation is co-administered with a long acting insulin such as insulin glargine or insulin degludec.

19. The formulation for use according to any one of claims 1 to 18, wherein the formulation is sufficiently stable that it remains substantially free of visible particles after storage at 25°C for at least 3 months and/or substantially free of visible particles after storage at 2-8°C for at least 9 months; and/or
wherein the formulation is sufficiently stable that it comprises no more than 1% (by weight of total protein) high molecular weight species after storage at 25°C for six months and/or the formulation of the invention comprises no more than 0.3% (by weight of total protein) high molecular weight species after storage at 2-8°C for twelve months; and/or
wherein the formulation is sufficiently stable that it comprises no more than 9% (by weight of total protein) of related species after storage at 25°C for six months and/or 5% (by weight of total protein) of related species after storage at 25°C for three months and/or the formulation of the invention comprises no more than 2% (by weight of total protein) of related species after storage at 2-8°C for twelve months.

## Patentansprüche

1. Wässrige, flüssige pharmazeutische Formulierung, umfassend (i) ein schnell wirkendes Insulinanalogon; (ii) ionisches Zink; und (iii) Citrat, wobei die Formulierung im Wesentlichen frei von einer zinkbindenden Spezies ist, die einen logK bezüglich der Zinkionenbindung von mehr als 12,3 bei 25 °C aufweist; zur Verwendung bei der Behandlung eines an Diabetes mellitus leidenden menschlichen Subjekts durch Verabreichung mittels subkutaner Injektion oder subkutaner Infusion als prandialer Bolus im Zeitfenster zwischen 20 Minuten nach Beginn einer Mahlzeit und 30 Minuten nach Beginn einer Mahlzeit,
wobei die Verabreichung von 0,3 U/kg der Formulierung zu Folgendem führt:
eine Zeit bis zum ersten Einsetzen der Insulinexposition (T_{INSONSET}) von höchstens 50 %;
des entsprechenden Wertes bei Verabreichung einer Formulierung, bestehend aus (i) Insulin aspart in einer Konzentration von 100 U/mL; (ii) Natriumphosphat in einer Konzentration von 8,8 mM; (iii) Natriumchlorid in einer Konzentration von 9,9 mM; (iv) Glycerol in einer Konzentration von 174 mM; (v) m-Cresol in einer Konzentration von 15,9 mM; (vi) Phenol in einer Konzentration von 15,9 mM; (vii) Argininhydrochlorid in einer Konzentration von 20 mM; (viii) Nicotinamid in einer Konzentration von 170 mM; (ix) ionischem Zink und (x) Wasser; und mit einem unter Verwendung von Natriumhydroxid auf 7,1 eingestellten pH-Wert.

2. Formulierung zur Verwendung nach Anspruch 1, wobei das schnell wirkende Insulinanalogon Insulin aspart ist.

3. Formulierung zur Verwendung nach Anspruch 1, wobei das schnell wirkende Insulinanalogon Insulin lispro ist.

4. Formulierung zur Verwendung nach einem der Ansprüche 1 bis 3, wobei das schnell wirkende Insulinanalogon in einer Konzentration von 50-200 U/ml vorliegt.

5. Formulierung zur Verwendung nach Anspruch 4, wobei das schnell wirkende Insulinanalogon in einer Konzentration von etwa 100 U/ml vorliegt.

6. Formulierung zur Verwendung nach einem der Ansprüche 1 bis 5, wobei das ionische Zink in einer Konzentration von 0,5-1 Gew.-% Zink, bezogen auf das Gewicht des schnell wirkenden Insulinanalogons in der Formulierung, vorliegt.

7. Formulierung zur Verwendung nach einem der Ansprüche 1 bis 6, wobei die Citratquelle Citronensäure ist.

8. Formulierung zur Verwendung nach einem der Ansprüche 1 bis 7, wobei das Citrat in einer Konzentration von 1-50 mM vorliegt.

9. Formulierung zur Verwendung nach einem der Ansprüche 1 bis 8, wobei das Molverhältnis von ionischem Zink zu Citrat 1:3 bis 1:175 beträgt.

10. Formulierung zur Verwendung nach einem der Ansprüche 1 bis 9, die im Wesentlichen frei von zinkbindenden Spezies ist, die einen logK bezüglich der Zinkionenbindung von 10-12,3 bei 25 °C aufweisen.

11. Formulierung zur Verwendung nach einem der Ansprüche 1 bis 10, wobei die Formulierung ferner ein nichtionisches Tensid umfasst, wobei das nichtionische Tensid ein Alkylglycosid ist, ausgewählt aus der Gruppe bestehend aus Dodecylmaltosid, Dodecylglucosid, Octylglucosid, Octylmaltosid, Decylglucosid, Decylmaltosid, Decylglucopyranosid, Tridecylglucosid, Tridecylmaltosid, Tetradecylglucosid, Tetradecylmaltosid, Hexadecylglucosid, Hexadecylmaltosid, Saccharosemonooctanoat, Saccharosemonodecanoat, Saccharosemonododecanoat, Saccharosemonotridecanoat, Saccharosemonotetradecanoat und Saccharosemonohexadecanoat; oder
wobei das nichtionische Tensid ein Polysorbat-Tensid ist; oder wobei das nichtionische Tensid ein Alkylether von Polyethylenglykol ist, ausgewählt aus Polyethylenglykol(2)-dodecylether, Polyethylenglykol(2)-oleylether und Polyethylenglykol(2)-hexadecylether; oder
wobei das nichtionische Tensid ein Blockcopolymer aus Polyethylenglykol und Polypropylenglykol ist, ausgewählt aus Poloxamer 188, Poloxamer 407, Poloxamer 171 oder Poloxamer 185;
oder
wobei das nichtionische Tensid ein Alkylphenylether von Polyethylenglykol ist;
wobei das nichtionische Tensid in einer Konzentration von 1-1000 µg/ml vorliegt.

12. Formulierung zur Verwendung nach Anspruch 11, wobei das nichtionische Tensid ein Alkylglycosid ist, ausgewählt aus der Gruppe bestehend aus Dodecylmaltosid und Decylglucopyranosid.

13. Formulierung zur Verwendung nach Anspruch 12, wobei das Alkylglycosid Dodecylmaltosid ist.

14. Formulierung zur Verwendung nach einem der Ansprüche 1 bis 13, ferner umfassend ein ungeladenes Tonizitätsmodifizierungsmittel, wobei das ungeladene Tonizitätsmodifizierungsmittel ausgewählt ist aus der Gruppe bestehend aus Trehalose, Mannitol, Glycerol und 1,2-Propandiol.

15. Formulierung zur Verwendung nach einem der Ansprüche 1 bis 13, ferner umfassend ein geladenes Tonizitätsmodifizierungsmittel in einer Konzentration von >60 mM, wobei das geladene Tonizitätsmodifizierungsmittel Natriumchlorid ist.

16. Formulierung zur Verwendung nach einem der Ansprüche 1 bis 15, wobei der pH-Wert im Bereich von 7,0 bis 7,8 liegt.

17. Formulierung zur Verwendung nach einem der Ansprüche 1 bis 16, ferner umfassend Nicotinamid; und/oder
ferner umfassend Nicotinsäure oder ein Salz davon; und/oder
ferner umfassend Treprostinil oder ein Salz davon; oder
wobei die Formulierung keinen Vasodilatator enthält.

18. Formulierung zur Verwendung nach einem der Ansprüche 1 bis 17, wobei die Formulierung ferner ein zusätzliches therapeutisch wirksames Mittel zur Verwendung bei der Behandlung von Diabetes umfasst, das ausgewählt ist aus der Gruppe bestehend aus einem Amylinanalogon und einem GLP-1-Agonisten; und/oder
wobei die Formulierung zusammen mit einem lang wirkenden Insulin wie Insulin glargin oder Insulin degludec verabreicht wird.

19. Formulierung zur Verwendung nach einem der Ansprüche 1 bis 18, wobei die Formulierung ausreichend stabil ist, dass sie nach Lagerung bei 25 °C für mindestens 3 Monate im Wesentlichen frei von sichtbaren Partikeln bleibt und/oder nach Lagerung bei 2-8 °C für mindestens 9 Monate im Wesentlichen frei von sichtbaren Partikeln ist; und/oder
wobei die Formulierung ausreichend stabil ist, dass sie nach sechsmonatiger Lagerung bei 25 °C nicht mehr als 1 % (bezogen auf das Gewicht des Gesamtproteins) hochmolekulare Spezies umfasst und/oder die erfindungsgemäße Formulierung nach zwölfmonatiger Lagerung bei 2-8 °C nicht mehr als 0,3 % (bezogen auf das Gewicht des Gesamtproteins) hochmolekulare Spezies umfasst; und/oder
wobei die Formulierung ausreichend stabil ist, dass sie nach sechsmonatiger Lagerung bei 25 °C nicht mehr als 9 % (bezogen auf das Gewicht des Gesamtproteins) verwandter Spezies und/oder nach dreimonatiger Lagerung bei 25 °C 5 % (bezogen auf das Gewicht des Gesamtproteins) verwandter Spezies umfasst und/oder die erfindungsgemäße Formulierung nach zwölfmonatiger Lagerung bei 2-8 °C nicht mehr als 2 % (bezogen auf das Gewicht des Gesamtproteins) verwandter Spezies umfasst.

## Revendications

1. Formulation pharmaceutique liquide aqueuse comprenant (i) un analogue d'insuline à action rapide ; (ii) du zinc ionique ; et (iii) du citrate, ladite formulation étant sensiblement exempte d'une espèce liant le zinc présentant un logK par rapport à la liaison à l'ion zinc supérieur à 12,3 à 25 °C ; pour une utilisation dans le traitement d'un sujet humain souffrant de diabète sucré par administration par injection sous-cutanée ou perfusion sous-cutanée sous forme de bolus prandial dans la fenêtre entre 20 minutes après le début d'un repas et 30 minutes après le début d'un repas,
dans laquelle l'administration de 0,3 U/kg de la formulation conduit à :
un délai jusqu'à l'apparition initiale de l'exposition à l'insuline (T_{INSONSET}) d'au plus 50 % ;
de la valeur correspondante lors de l'administration d'une formulation constituée (i) d'insuline asparte à une concentration de 100 U/ml ; (ii) de phosphate de sodium à une concentration de 8,8 mM ; (iii) de chlorure de sodium à une concentration de 9,9 mM ; (iv) de glycérol à une concentration de 174 mM ; (v) de m-crésol à une concentration de 15,9 mM ; (vi) de phénol à une concentration de 15,9 mM ; (vii) de chlorhydrate d'arginine à une concentration de 20 mM ; (viii) de nicotinamide à une concentration de 170 mM ; (ix) de zinc ionique et (x) d'eau ; et avec un pH ajusté à 7,1 à l'aide d'hydroxyde de sodium.

2. Formulation pour une utilisation selon la revendication 1, dans laquelle l'analogue d'insuline à action rapide est l'insuline asparte.

3. Formulation pour une utilisation selon la revendication 1, dans laquelle l'analogue d'insuline à action rapide est l'insuline lispro.

4. Formulation pour une utilisation selon l'une quelconque des revendications 1 à 3, dans laquelle l'analogue d'insuline à action rapide est présent à une concentration de 50 à 200 U/ml.

5. Formulation pour une utilisation selon la revendication 4, dans laquelle l'analogue d'insuline à action rapide est présent à une concentration d'environ 100 U/ml.

6. Formulation pour une utilisation selon l'une quelconque des revendications 1 à 5, dans laquelle le zinc ionique est présent à une concentration de 0,5 à 1 % en poids de zinc par rapport au poids d'analogue d'insuline à action rapide dans la formulation.

7. Formulation pour une utilisation selon l'une quelconque des revendications 1 à 6, dans laquelle la source du citrate est l'acide citrique.

8. Formulation pour une utilisation selon l'une quelconque des revendications 1 à 7, dans laquelle le citrate est présent à une concentration de 1 à 50 mM.

9. Formulation pour une utilisation selon l'une quelconque des revendications 1 à 8, dans laquelle le rapport molaire du zinc ionique au citrate est de 1:3 à 1:175.

10. Formulation pour une utilisation selon l'une quelconque des revendications 1 à 9, qui est sensiblement exempte d'espèces liant le zinc présentant un logK par rapport à la liaison à l'ion zinc de 10 à 12,3 à 25 °C.

11. Formulation pour une utilisation selon l'une quelconque des revendications 1 à 10, dans laquelle la formulation comprend en outre un tensioactif non ionique, dans laquelle le tensioactif non ionique est un alkylglycoside choisi dans le groupe constitué du dodécylmaltoside, du dodécylglucoside, de l'octylglucoside, de l'octylmaltoside, du décylglucoside, du décylmaltoside, du décylglucopyranoside, du tridécylglucoside, du tridécylmaltoside, du tétradécylglucoside, du tétradécylmaltoside, de l'hexadécylglucoside, de l'hexadécylmaltoside, du monooctanoate de saccharose, du monodécanoate de saccharose, du monododécanoate de saccharose, du monotridécanoate de saccharose, du monotétradécanoate de saccharose et du monohexadécanoate de saccharose ; ou
dans laquelle le tensioactif non ionique est un tensioactif de type polysorbate ; ou
dans laquelle le tensioactif non ionique est un éther alkylique de polyéthylène glycol choisi parmi l'éther dodécylique de polyéthylène glycol (2), l'éther oléylique de polyéthylène glycol (2) et l'éther hexadécylique de polyéthylène glycol (2) ; ou
dans laquelle le tensioactif non ionique est un copolymère à blocs de polyéthylène glycol et de polypropylène glycol choisi parmi le poloxamère 188, le poloxamère 407, le poloxamère 171 ou le poloxamère 185 ; ou
dans laquelle le tensioactif non ionique est un éther d'alkylphényle de polyéthylène glycol ;
dans laquelle le tensioactif non ionique est présent à une concentration de 1 à 1000 µg/ml.

12. Formulation pour une utilisation selon la revendication 11, dans laquelle le tensioactif non ionique est un alkylglycoside choisi dans le groupe constitué du dodécylmaltoside et du décylglucopyranoside.

13. Formulation pour une utilisation selon la revendication 12, dans laquelle l'alkylglycoside est le dodécylmaltoside.

14. Formulation pour une utilisation selon l'une quelconque des revendications 1 à 13, comprenant en outre un agent modificateur de tonicité non chargé, dans laquelle l'agent modificateur de tonicité non chargé est choisi dans le groupe constitué du tréhalose, du mannitol, du glycérol et du 1,2-propanediol.

15. Formulation pour une utilisation selon l'une quelconque des revendications 1 à 13, comprenant en outre un agent modificateur de tonicité chargé présent à une concentration > 60 mM, dans laquelle l'agent modificateur de tonicité chargé est le chlorure de sodium.

16. Formulation pour une utilisation selon l'une quelconque des revendications 1 à 15, dans laquelle le pH est situé dans la plage de 7,0 à 7,8.

17. Formulation pour une utilisation selon l'une quelconque des revendications 1 à 16, comprenant en outre du nicotinamide ; et/ou
comprenant en outre de l'acide nicotinique ou un sel de celui-ci ; et/ou
comprenant en outre du tréprostinil ou un sel de celui-ci ; ou
dans laquelle la formulation ne contient pas de vasodilatateur.

18. Formulation pour une utilisation selon l'une quelconque des revendications 1 à 17, dans laquelle la formulation comprend en outre un agent thérapeutiquement actif supplémentaire utile dans le traitement du diabète choisi dans le groupe constitué d'un analogue de l'amyline et d'un agoniste du GLP-1 ; et/ou
dans laquelle la formulation est co-administrée avec une insuline à action prolongée telle que l'insuline glargine ou l'insuline dégludec.

19. Formulation pour une utilisation selon l'une quelconque des revendications 1 à 18, dans laquelle la formulation est suffisamment stable pour rester sensiblement exempte de particules visibles après conservation à 25 °C pendant au moins 3 mois et/ou sensiblement exempte de particules visibles après conservation entre 2 et 8 °C pendant au moins 9 mois ; et/ou
dans laquelle la formulation est suffisamment stable pour ne pas comprendre plus de 1 % (en poids des protéines totales) d'espèces de haut poids moléculaire après conservation à 25 °C pendant six mois et/ou la formulation selon l'invention ne comprend pas plus de 0,3 % (en poids des protéines totales) d'espèces de haut poids moléculaire après conservation entre 2 et 8 °C pendant douze mois ; et/ou
dans laquelle la formulation est suffisamment stable pour ne pas comprendre plus de 9 % (en poids des protéines totales) d'espèces apparentées après conservation à 25 °C pendant six mois et/ou 5 % (en poids des protéines totales) d'espèces apparentées après conservation à 25 °C pendant trois mois et/ou la formulation selon l'invention ne comprend pas plus de 2 % (en poids des protéines totales) d'espèces apparentées après conservation entre 2 et 8 °C pendant douze mois.
